# EUROPEAN PATENT SPECIFICATION

(11) **EP 4 459 235 B1**
(45) Date of publication and mention of the grant of the patent: **22.04.2026**
(21) Application number: 22933367.9
(22) Date of filing: 24.03.2022
(51) Int. Cl.: G01D 5/26, G01N 21/41, G01N 21/25, G01N 21/45, G01N 21/77

(54) **OPTICAL SENSOR DEVICE, MEASUREMENT SYSTEM, AND MEASUREMENT METHOD**
OPTISCHE SENSORVORRICHTUNG, MESSSYSTEM UND MESSVERFAHREN
DISPOSITIF DE CAPTEUR OPTIQUE, SYSTÈME DE MESURE ET PROCÉDÉ DE MESURE

(43) Date of publication of application: 06.11.2024
(73) Proprietor: MITSUBISHI ELECTRIC CORPORATION, Chiyoda-ku Tokyo 100-8310 (JP)
(72) Inventor: FUJIE, Akihiro, Tokyo 100-8310 (JP); OTSUKA, Hiroshi, Tokyo 100-8310 (JP); ONO, Hitomi, Tokyo 100-8310 (JP); ANDO, Toshiyuki, Tokyo 100-8310 (JP)
(74) Representative: Pfenning, Meinig & Partner mbB
(86) International application number: PCT/JP2022/013781
(87) International publication number: WO 2023/181226

(56) References cited:
- EP-B1- 3 013 217
- JP-A- 2003 527 625
- JP-A- 2015 502 539
- JP-A- 2022 506 275
- US-A1- 2008 132 808
- US-A1- 2014 321 502
- US-A1- 2019 234 850
- US-A1- 2020 386 718
- US-B2- 9 797 795
- SINGHA SATYABRATA; BHOWMIK BISHANKA BRATA: "On-chip Photonic Temperature Sensor Using Micro Ring Resonator", 2018 FIFTEENTH INTERNATIONAL CONFERENCE ON WIRELESS AND OPTICAL COMMUNICATIONS NETWORKS (WOCN), IEEE, 2 February 2018 (2018-02-02), pages 1 - 4, XP033460756, DOI: 10.1109/WOCN.2018.8556121

## Description

### TECHNICAL FIELD

The present disclosure relates to an optical sensor device, a measurement system, and a measurement method.

### BACKGROUND ART

In recent years, the technique of nondestructively measuring the state of a specimen has become widespread. For example, Patent Literature 1 describes a sensor that nondestructively measures a magnetic field based on a pulse signal of a specimen. This sensor includes a plurality of magnetic field sensors, and includes a sensor circuit unit for each magnetic field sensor. A magnetic-field detection signal is output from the magnetic field sensor to the sensor circuit unit via an electric wire.

US 9 797 795 B2 describes a pressure sensing pad with a flexible planar layer having a two-dimensional pressure sensing area. An optical fiber is attached to and traverses the pressure sensing area of the flexible planar layer.

US 2008/132808 A1 describes a bed occupant monitoring system comprising a pressure sensitive member attached to a support member for supporting a bed occupant.

EP 3 013 217 B1 describes a device for optical detection of analytes in a sample.

### CITATION LIST

### PATENT LITERATURES

Patent Literature 1: JP 2017-223570 A. Patent documents US 9 797 795 B2, US 2008/132808 A2, EP 3 013 217 B1.

### SUMMARY OF INVENTION

### TECHNICAL PROBLEM

In the sensor described in Patent Literature 1, the magnetic-field detection signal output from the magnetic field sensor is an electric signal, and the sensor circuit unit that measures the magnetic field of a specimen using the electric signal is provided for each magnetic field sensor. Therefore, the sensor described in Patent Literature 1 has a problem that power consumption is large.

The present disclosure solves the above problems, and an object thereof is to obtain an optical sensor device, a measurement system, and a measurement method capable of detecting the state of a specimen without using an electric signal for a specimen-state detection signal and without providing a sensor circuit unit for each sensor chip.

### SOLUTION TO PROBLEM

The invention is set out in the appended set of claims.

### ADVANTAGEOUS EFFECTS OF INVENTION

According to the present disclosure, the optical sensor device includes: a plurality of optical sensor chips that have one or a plurality of changer units to change a characteristic of an input optical signal depending on a state of a specimen, and that output an optical signal with a changed characteristic; an optical fiber that connects among the plurality of optical sensor chips through which an optical signal propagates, and via which the optical signal is input to and output from the plurality of optical sensor chips; and a chip holding member to which the plurality of optical sensor chips are provided. Since the optical signal is input and output between the optical sensor chips through the optical fiber, the optical sensor device according to the present disclosure can detect the state of the specimen without using an electric signal for a specimen-state detection signal and without providing a sensor circuit unit for each sensor chip.

### BRIEF DESCRIPTION OF DRAWINGS

FIG. 1 is a configuration diagram illustrating a measurement system according to a first embodiment.
FIG. 2A is a cross-sectional arrow view illustrating a cross-section of an optical sensor sheet taken along line A-A in FIG. 1, and FIG. 2B is a cross-sectional arrow view illustrating a cross-section of the optical sensor sheet on which a specimen is disposed taken along line A-A in FIG. 1.
FIG. 3 is a block diagram illustrating a configuration of the optical sensor sheet.
FIG. 4 is a flowchart illustrating a measurement method according to the first embodiment.
FIG. 5 is a flowchart illustrating details of the measurement method according to the first embodiment.
FIG. 6 is a configuration diagram illustrating a first modification of a changer unit.
FIG. 7 is a flowchart illustrating an operation of an optical sensor chip including the changer unit of FIG. 6.
FIG. 8 is a graph illustrating a relationship between the wavelength and the intensity of an optical signal propagating through the changer unit included in the optical sensor chip of the optical sensor sheet on which the specimen is not disposed.
FIG. 9 is a graph illustrating a relationship between the wavelength and the intensity of an optical signal propagating through the changer unit included in the optical sensor chip of the optical sensor sheet on which the specimen is disposed.
FIG. 10 is a graph illustrating a relationship between the position of the optical sensor chip of the optical sensor sheet on which the specimen is disposed and the moisture content of the specimen.
FIG. 11 is a block diagram illustrating a first configuration example of a measurement device.
FIG. 12 is a configuration diagram illustrating a first modification of the measurement system according to the first embodiment.
FIG. 13 is a configuration diagram illustrating a second modification of the measurement system according to the first embodiment.
FIG. 14 is a configuration diagram illustrating a second modification of the changer unit.
FIG. 15 is a configuration diagram illustrating a third modification of the changer unit.
FIG. 16 is a block diagram illustrating a second configuration example of the measurement device.
FIG. 17 is a block diagram illustrating a third configuration example of the measurement device.
FIG. 18A is a block diagram illustrating a hardware configuration for implementing the functions of the measurement device, and FIG. 18B is a block diagram illustrating a hardware configuration for executing software for implementing the functions of the measurement device.
FIG. 19 is a configuration diagram illustrating a measurement system according to a second embodiment.
FIG. 20 is a block diagram illustrating a configuration of a measurement device according to a third embodiment.

### DESCRIPTION OF EMBODIMENTS

### First Embodiment

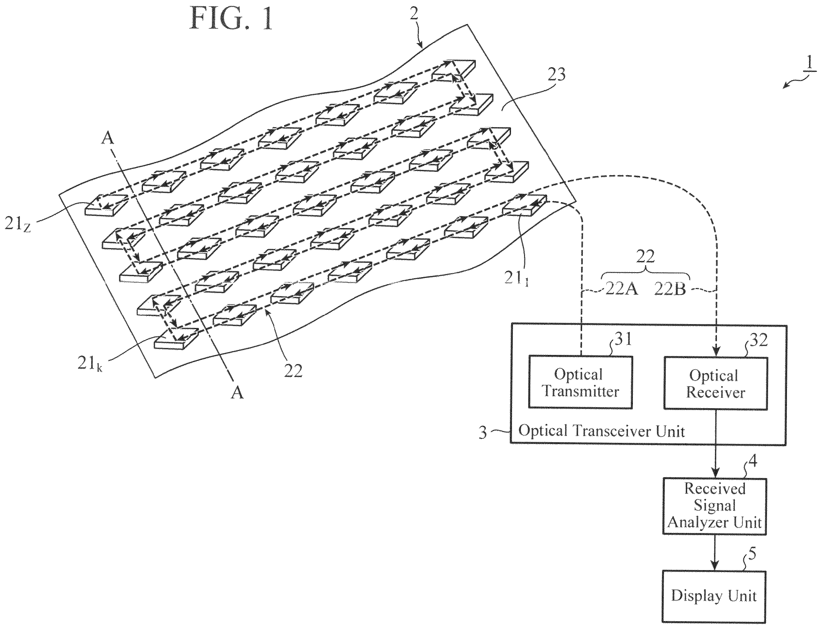

FIG. 1 is a configuration diagram illustrating a measurement system 1 according to a first embodiment. In FIG. 1, the measurement system 1 is a system that measures the state of a specimen using an optical sensor sheet 2. The measurement system 1 can measure the state of a solid, liquid, or gas specimen, and the specimen may be a living body such as a human or an animal. The state of the specimen is a state where optical measurement by an optical sensor chip 21ₖ provided in the optical sensor sheet 2 can be performed, and is a state inside the specimen or a state around the specimen. The state of the specimen is, for example, a temperature inside or around the specimen, a moisture concentration of the specimen, or a pressure applied from the specimen.

The optical sensor sheet 2 is an optical sensor device including a plurality of the optical sensor chips 21ₖ, an optical fiber 22, and a sheet member 23, wherein "k" is any natural number equal to or more than 1 and equal to or less than Z, and in FIG. 1, the optical sensor sheet 2 includes Z optical sensor chips 21ₖ.

The sheet member 23 is a chip holding member including Z optical sensor chips 21ₖ from an optical sensor chip 21₁ to an optical sensor chip 21_{Z} via the optical fiber 22. For example, the sheet member 23 is a planar insulating sheet. In the following description, it is assumed that the sheet member 23 is a thin and soft member that is used by being laid under the specimen or by covering the specimen, but the sheet member 23 may be a rigid plate-like member.

In addition to the planar insulating sheet, the sheet member 23 may be a deformable tin plate or a bath lid with a fixed shape.

The Z optical sensor chips 21ₖ are continuously arranged and wired from the optical sensor chip 21₁ to the optical sensor chip 21_{Z} on the sheet member 23.

Furthermore, in the Z optical sensor chips 21ₖ, other optical sensor chips 21ₖ may be arranged and wired radially from one optical sensor chip 21ₖ.

The optical sensor device according to the first embodiment may be a device in which the sheet member 23 is not provided, and Z optical sensor chips 21ₖ from the optical sensor chip 21₁ to the optical sensor chip 21_{Z} are continuously connected via the optical fiber 22 on the surface of the specimen or the surface on which the specimen is disposed. Even in the optical sensor device configured as described above, since an optical signal is input and output between the optical sensor chips through the optical fiber 22, it is possible to detect the state of the specimen without using an electric signal for a specimen-state detection signal and without providing a sensor circuit unit for each sensor chip.

According to the invention the optical fiber 22 includes a transmission optical fiber 22A and a reception optical fiber 22B. The optical fiber 22 may be attached to or embedded in the sheet member 23. In addition, the optical fiber 22 may be one optical fiber for both transmission and reception in an example not being part of the invention.

In FIG. 1, the sheet member 23 has a rectangular shape, and the plurality of continuous optical sensor chips 21ₖ are arranged in a meandering state in such a manner as to be housed in the sheet member 23. Note that the shape of the sheet member 23 is not limited to a rectangle, and may be a triangle, a polygon with five or more corners, or a circle. Furthermore, the shape of the sheet member 23 may be a shape conforming to the outer shape of the specimen. For example, in a case where the specimen has an elongated outer shape, the sheet member 23 has an elongated shape.

Among the Z optical sensor chips 21ₖ continuously connected in the optical sensor sheet 2, the optical sensor chip 21₁ at one end is a first optical sensor chip that receives and outputs an optical signal to and from an optical transceiver unit 3. The optical fiber 22A and the optical fiber 22B are optical paths that optically connect the optical sensor chip 21₁ and the optical transceiver unit 3.

The optical transceiver unit 3 includes an optical transmitter 31 and an optical receiver 32. The optical transmitter 31 transmits an optical signal to the optical sensor chip 21₁ through the optical fiber 22A. The optical receiver 32 receives an optical signal from the optical sensor chip 21₁ through the optical fiber 22B. The optical transmitter 31 outputs light emitted from a light emitting element as a light source to the optical fiber 22A.

Furthermore, the optical transmitter 31 and the optical receiver 32 may be individually wired to each optical sensor chip 21ₖ.

The optical receiver 32 acquires an electrical signal converted from an optical signal by a light receiving element. The light emitting element and the light receiving element may be provided separately, or may be an optical sensor in which the light emitting element and the light receiving element are integrated into one. The optical transceiver unit 3 may be provided in a measurement device provided separately from the optical sensor sheet 2, or may be provided in the optical sensor sheet 2. For example, the optical transceiver unit 3 may be integrated on an InP substrate provided on the optical sensor sheet 2.

Among the Z optical sensor chips 21ₖ continuously connected in the optical sensor sheet 2, the optical sensor chip 21_{Z} at the other end is a second optical sensor chip that changes the characteristics of an optical signal output from an optical sensor chip at the preceding stage, and outputs the optical signal with the changed characteristics to an optical sensor chip at the preceding stage by return.

The optical sensor chip 21_{Z} turns the optical signal sequentially propagated through the plurality of optical sensor chips 21ₖ that are continuous between the optical sensor chip 21₁ and the optical sensor chip 21_{Z} toward the optical sensor chip 21₁. The optical signal turned at the optical sensor chip 21_{Z} is sequentially propagated through the plurality of optical sensor chips that are continuous between the optical sensor chip 21₁ and the optical sensor chip 21_{Z} again, and is output from the optical sensor chip 21₁ to the optical transceiver unit 3.

A received signal analyzer unit 4 measures the state of the specimen by analyzing the optical signal received from the optical sensor chip 21₁ by the optical transceiver unit 3. A display unit 5 receives measurement results of the state of the specimen from the received signal analyzer unit 4, and displays the received measurement results on a display included in the measurement device.

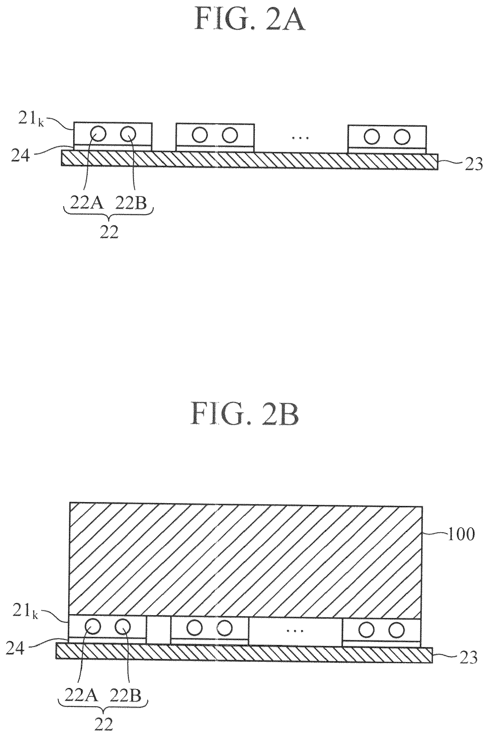

FIG. 2A is a cross-sectional arrow view illustrating a cross-section of the optical sensor sheet 2 taken along line A-A in FIG. 1. In addition, FIG. 2B is a cross-sectional arrow view illustrating a cross-section of the optical sensor sheet 2 on which a specimen 100 is disposed taken along line A-A in FIG. 1. As illustrated in FIGS. 2A and 2B, each of the plurality of optical sensor chips 21ₖ is attached to the sheet member 23 by an adhesive layer 24. The optical sensor chips 21ₖ adjacent to each other in the direction in which the plurality of optical sensor chips 21ₖ are continuous are connected by the optical fiber 22A and the optical fiber 22B.

In the optical sensor sheet 2, as illustrated in FIG. 2B, the specimen 100 is disposed on the optical sensor chip 21ₖ. The optical sensor chip 21ₖ includes, for example, an optical waveguide manufactured using microfabrication technology represented by silicon photonics technology. The optical sensor chip 21ₖ changes at least one of the intensity, the phase, or the frequency of an optical signal depending on the state of the specimen 100.

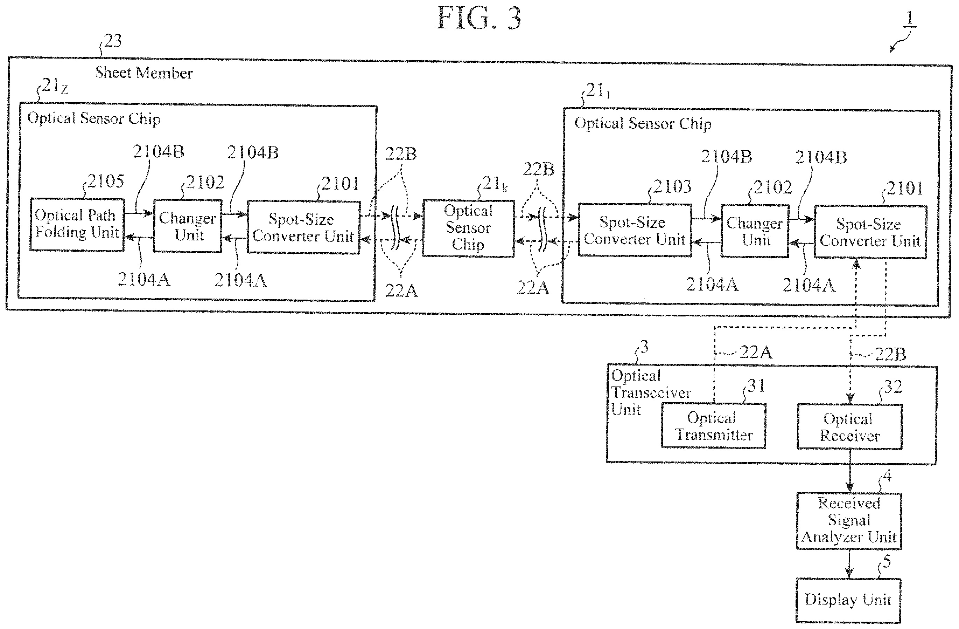

FIG. 3 is a block diagram illustrating a configuration of the optical sensor sheet 2. In FIG. 3, Z optical sensor chips 21ₖ are continuously connected in the optical sensor sheet 2. Among the Z optical sensor chips 21ₖ, optical sensor chips from the optical sensor chip 21₁ to an optical sensor chip 21_{Z-1} each include a spot-size converter unit 2101, a changer unit 2102, a spot-size converter unit 2103, a waveguide 2104A, and a waveguide 2104B. In addition, the optical sensor chip 21_{Z} includes the spot-size converter unit 2101, the changer unit 2102, the waveguide 2104A, the waveguide 2104B, and an optical path folding unit 2105.

The spot-size converter unit 2101 and the spot-size converter unit 2103 are optical elements that convert the spot size, which is the magnitude of the spread of the light distribution in the optical fiber, and the spot size in the waveguide. The spot-size converter unit 2101 and the spot-size converter unit 2103 are implemented by, for example, a spot size converter including a waveguide. The waveguide is, for example, a silicon waveguide.

The spot-size converter unit 2101 converts the spot size of an optical signal propagated through the optical fiber 22A on the basis of the waveguide 2104A, and outputs the optical signal with the converted spot size to the waveguide 2104A. In addition, the spot-size converter unit 2101 converts the spot size of an optical signal propagated through the waveguide 2104B on the basis of the optical fiber 22B, and outputs the optical signal with the converted spot size to the optical fiber 22B.

The spot-size converter unit 2103 converts the spot size of an optical signal propagated through the waveguide 2104A on the basis of the optical fiber 22A, and outputs the optical signal with the converted spot size to the optical fiber 22A. In addition, the spot-size converter unit 2103 converts the spot size of an optical signal propagated through the optical fiber 22B on the basis of the waveguide 2104B, and outputs the optical signal with the converted spot size to the waveguide 2104B.

The changer unit 2102 changes the characteristics of the input optical signal depending on the state of the specimen 100, and outputs the optical signal with the changed characteristics to the outside. The characteristics of the optical signal include an intensity characteristic, a phase characteristic, or a frequency characteristic of the optical signal. The changer unit 2102 may change at least one of the intensity characteristic, the phase characteristic, or the frequency characteristic of the input optical signal.

In addition, the changer unit 2102 is implemented by, for example, a ring resonator including an optical waveguide. It is not limited to the ring resonator, and the changer unit 2102 is only required to change the characteristics of the optical signal depending on the state of the specimen 100, and may be a phase shifter including an optical waveguide, a frequency shifter including an optical waveguide, or an optical element including a combination of the phase shifter and the frequency shifter.

Furthermore, the changer unit 2102 may be a ring resonator, a Mach-Zehnder interferometer (MZI), or a combination thereof. Even the changer unit 2102 configured as described above can change the characteristics of the optical signal depending on the state of the specimen 100.

Furthermore, a plurality of the changer units 2102 may be provided in one optical sensor chip, and the plurality of changer units 2102 may change the characteristics of the input optical signal depending on a plurality of mutually different states of the specimen 100. For example, among the plurality of changer units 2102 connected in series, a certain changer unit 2102 changes the characteristics of the input optical signal depending on the temperature of the specimen 100, another changer unit 2102 changes the characteristics of the input optical signal depending on the moisture concentration of the specimen 100, and still another changer unit 2102 changes the characteristics of the input optical signal depending on the pressure applied from the specimen 100. The optical sensor chip configured as described above can change the characteristics of the input optical signal depending on a plurality of mutually different states of the specimen 100.

The waveguide 2104A and the waveguide 2104B are thin wire waveguides that optically connect the spot-size converter unit 2101 and the changer unit 2102, and propagate an optical signal with the converted spot size. For example, the waveguide 2104A propagates an optical signal from the optical sensor chip 21₁ to the optical sensor chip 21_{Z}. The waveguide 2104B propagates an optical signal from the optical sensor chip 21_{Z} to the optical sensor chip 21₁.

The optical path folding unit 2105 is a reflector unit for reflecting the optical signal whose characteristics have been changed by the changer unit 2102 to the optical sensor chip 21_{Z-1} at the preceding stage. For example, the optical path folding unit 2105 is implemented by a loop mirror. The optical signal reflected by the optical path folding unit 2105 is sequentially propagated through Z optical sensor chips 21ₖ continuously connected in the optical sensor sheet 2, and is output from the optical sensor chip 21₁ to the optical transceiver unit 3.

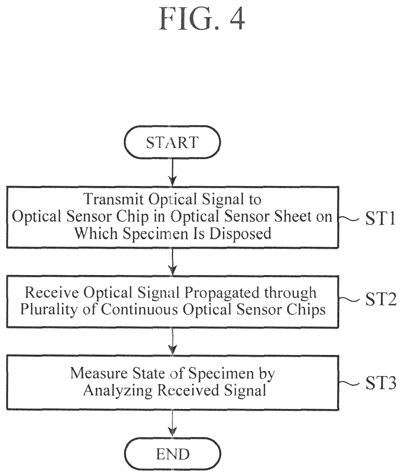

FIG. 4 is a flowchart illustrating a measurement method according to the first embodiment, and illustrates the measurement method of the state of the specimen 100 using the optical sensor sheet 2. The optical transceiver unit 3 transmits an optical signal to the optical sensor chip 21₁ in the optical sensor sheet 2 on which the specimen 100 is disposed (step ST1). The optical transceiver unit 3 receives an optical signal that has reciprocated between Z optical sensor chips 21ₖ from the optical sensor chip 21₁, and outputs the received signal to the received signal analyzer unit 4 (step ST2). The received signal analyzer unit 4 measures the state of the specimen 100 by analyzing the signal input from the optical transceiver unit 3 (step ST3).

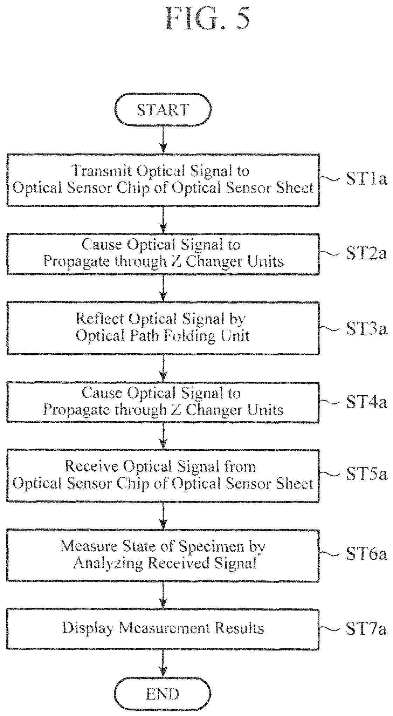

FIG. 5 is a flowchart illustrating details of the measurement method according to the first embodiment. In the following description, it is assumed that the optical transceiver unit 3, the received signal analyzer unit 4, and the display unit 5 are included in a measurement device provided separately from the optical sensor sheet 2. Hereinafter, the display of information by the display unit 5 means that information is displayed on a display included in the measurement device.

First, the optical transmitter 31 included in the optical transceiver unit 3 generates an optical signal using light emitted from a light source and transmits the generated optical signal to the optical sensor chip 21₁ (step ST1a). The transmission of the optical signal means that the optical signal is output from the optical transmitter 31 to the optical fiber 22A, and the optical sensor chip 21₁ receives the optical signal propagated through the optical fiber 22A.

For example, in a case where moisture contained in the specimen 100 is detected, a wavelength in the absorption wavelength band of water is set in each of the plurality of optical sensor chips 21ₖ. The optical transmitter 31 transmits an optical pulse signal in which Z wavelengths set in the Z optical sensor chips 21ₖ are multiplexed to the optical sensor chip 21₁.

The optical signal input to the optical sensor chip 21₁ sequentially propagates through the changer unit 2102 included in each of the optical sensor chips 21ₖ from the optical sensor chip 21₁ to the optical sensor chip 21_{Z} (step ST2a). When the optical signal reaches the optical sensor chip 21_{Z}, the optical signal is reflected to the side of the optical sensor chip 21₁ by the optical path folding unit 2105 (step ST3a). As a result, the reflected optical signal sequentially propagates through the changer unit 2102 included in each of the optical sensor chips 21ₖ from the optical sensor chip 21_{Z} to the optical sensor chip 21₁ (step ST4a).

While the optical signal sequentially propagates through the Z optical sensor chips 21ₖ, the characteristics of the optical signal are changed depending on the state of the specimen 100 in the changer unit 2102 included in each of the optical sensor chips 21ₖ. For example, in a case where the wavelength in the absorption wavelength band of water is set as the resonance wavelength in the changer unit 2102, in the changer unit 2102, the optical signal component having the wavelength in the absorption wavelength band of water in the wavelength-multiplexed optical signal is absorbed by water present inside or around the specimen 100, and the intensity thereof changes.

The optical receiver 32 included in the optical transceiver unit 3 receives an optical signal from the optical sensor chip 21₁ (step ST5a). Here, the reception of the optical signal means that the optical signal output from the optical sensor chip 21₁ to the optical fiber 22B is converted into an electrical signal by the light receiving element, and the electrical signal is input to the optical receiver 32. The received signal analyzer unit 4 measures the state of the specimen 100 by analyzing the signal input from the optical transceiver unit 3 (step ST6a).

For example, the received signal analyzer unit 4 calculates a reciprocating propagation time of a pulse using the pulse of a transmitted optical signal and the pulse of an input optical signal, and specifies the position of the optical sensor chip 21ₖ on the basis of the reciprocating propagation time. The received signal analyzer unit 4 calculates the difference between pulse peak values of the pulse of the transmitted optical signal and the pulse of the input optical signal, and measures a moisture content inside or around the specimen 100 for each optical sensor chip 21ₖ using a value of the calculated difference. For example, the intensity of the optical signal decreases in proportion to the moisture content. The difference between the pulse peak values of the pulse of the transmitted optical signal and the pulse of the input optical signal is the amount of change in the intensity of the optical signal component with a wavelength assigned to the optical sensor chip 21ₖ.

The display unit 5 receives measurement results of the state of the specimen from the received signal analyzer unit 4, and displays the received measurement results (step ST7a). For example, the received signal analyzer unit 4 can measure the two-dimensional distribution of the moisture content in the specimen 100 by specifying the position of the optical sensor chip 21ₖ on the optical sensor sheet 2. As a result, the display unit 5 can graphically display the two-dimensional distribution of the moisture content of the specimen 100.

For example, in the case of detecting the pressure from the specimen 100, a different resonance frequency is set in each of the plurality of optical sensor chips 21ₖ. That is, the optical sensor chip 21ₖ is configured to resonate at an individually set frequency. When a pressure is applied from the specimen 100 to the waveguide of the optical sensor chip 21ₖ, the waveguide is distorted and resonance conditions change. As a result, the ratio of the optical signal resonated at the frequency set in the optical sensor chip 21ₖ changes. By analyzing the change in the ratio, it is possible to detect the pressure from the specimen 100 for each optical sensor chip. In addition, the two-dimensional distribution of the pressure from the specimen 100 can be measured by specifying the position of the optical sensor chip 21ₖ on the optical sensor sheet 2. As a result, the display unit 5 can graphically display the two-dimensional distribution of the pressure from the specimen 100.

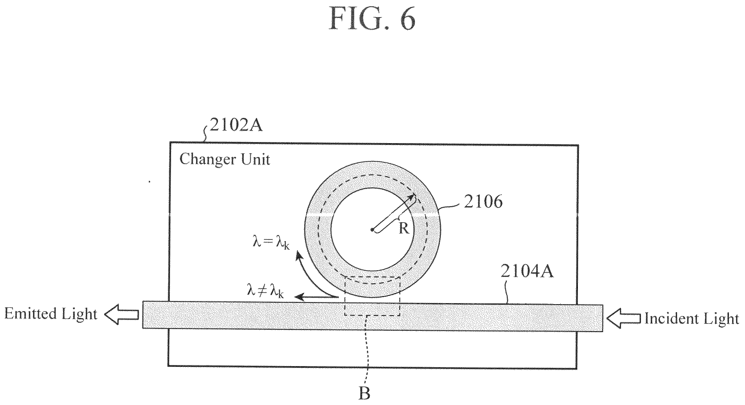

FIG. 6 is a configuration diagram illustrating a changer unit 2102A that is a first modification of the changer unit 2102. The changer unit 2102A is a ring resonator. As illustrated in FIG. 6, the ring resonator includes a waveguide 2106 with a ring shape. A resonance wavelength based on a curvature radius R and a waveguide effective refractive index n_{eff} of the waveguide 2106 is set in the changer unit 2102A. The waveguide effective refractive index n_{eff} can be changed by disposing a micro-heater on the waveguide 2106 or stacking a different magnetic body other than silicon.

The changer unit 2102A satisfies the relationship of 2π × R × n_{eff} = m × λₖ, wherein "m" is an integer, and λₖ is a resonance wavelength set in the changer unit 2102A included in the optical sensor chip 21ₖ. A different resonance wavelength λₖ is set in each of the changer units 2102A individually included in the Z optical sensor chips 21ₖ. The setting of the resonance wavelength λₖ means that the waveguide 2106 is configured with the curvature radius R and the waveguide effective refractive index n_{eff} that cause it to resonate at the wavelength λₖ.

For example, in a case where moisture inside or around the specimen 100 is detected using the optical sensor sheet 2, the wavelength λₖ in the absorption wavelength band of water is set in the changer unit 2102A included in each of the Z optical sensor chips 21ₖ. That is, the wavelength λ₁ is set in the changer unit 2102A included in the optical sensor chip 21₁, the wavelength λₖ is set in the changer unit 2102A included in the optical sensor chip 21ₖ, and the wavelength λ_{Z} is set in the changer unit 2102A included in the optical sensor chip 21_{Z}.

A region B surrounded by a broken line in FIG. 6 is a region where the waveguide 2104A and the waveguide 2106 are close to each other. Of the optical signal incident on the changer unit 2102A, the optical signal component with the wavelength λₖ propagates through the waveguide 2106 in the region B, and the optical signal component with the wavelength λ (≠ λₖ) other than the wavelength λₖ directly propagates through the waveguide 2104A and is emitted from the changer unit 2102A.

The optical signal with the wavelength λₖ resonates while circulating in the waveguide 2106, and is transmitted to the specimen 100 disposed on the waveguide 2106 or to the periphery of the specimen 100 close to the waveguide 2106 during the resonance. In a case where water is present inside the specimen 100 or around the specimen 100, the transmitted optical signal component with the wavelength λₖ is absorbed by the water, so that the intensity of the optical signal with the wavelength λₖ decreases in proportion to the moisture content. As a result, the received signal analyzer unit 4 can measure the moisture content of the specimen 100 by analyzing the change in the intensity of the optical signal.

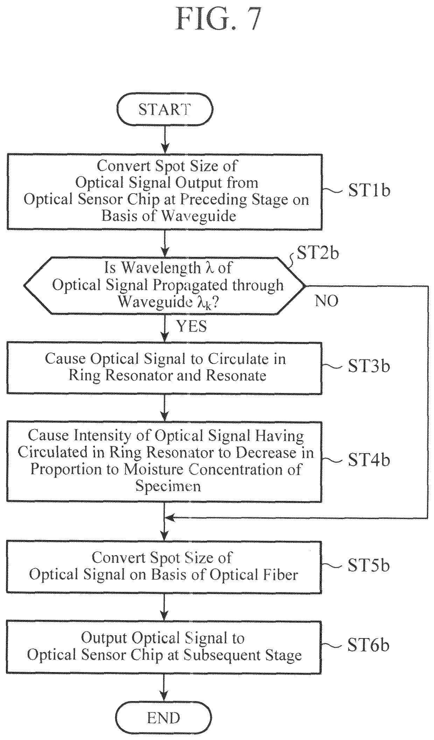

FIG. 7 is a flowchart illustrating an operation of the optical sensor chip 21ₖ including the changer unit 2102A, and illustrates a case where moisture inside or around the specimen 100 is detected using the optical sensor sheet 2. The optical transceiver unit 3 transmits an optical signal in which the wavelengths λ₁ to λ_{Z} set in Z optical sensor chips 21ₖ are multiplexed to the optical sensor chip 21₁. The optical signal transmitted to the optical sensor chip 21₁ propagates sequentially from the optical sensor chip 21₁ to the optical sensor chip 21_{Z}. The spot-size converter unit 2101 included in the optical sensor chip 21ₖ converts the spot size of the optical signal output from the optical sensor chip at the preceding stage on the basis of the waveguide 2104A (step ST1b).

The optical signal having the spot size converted and having propagated through the waveguide 2104A is incident on the changer unit 2102A. For example, the waveguide 2106, which is a ring resonator, specifies whether or not the optical signal incident on the changer unit 2102A is a signal component with the wavelength λₖ and extracts the signal component (step ST2b). When the signal component with the wavelength λₖ is extracted from the optical signal incident on the changer unit 2102A in the region B (step ST2b; YES), the extracted optical signal component circulates in the waveguide 2106 and resonates (step ST3b).

While the signal component circulates in the ring resonator, the intensity of the signal component with the wavelength λₖ decreases in proportion to the moisture concentration (step ST4b). The signal component having circulated in the ring resonator reflects to the waveguide 2104A again and is emitted from the changer unit 2102A. In addition, in the region B, the signal component with the wavelength λ other than the wavelength λₖ is not extracted (step ST2b; NO) and the signal component with the wavelength λ (≠ λₖ) directly propagates through the waveguide 2104A and is emitted from the changer unit 2102A.

The spot-size converter unit 2103 converts the spot size of the optical signal emitted from the changer unit 2102A on the basis of the optical fiber 22A (step ST5b). The optical signal with the converted spot size is output to the optical sensor chip 21ₖ at the subsequent stage through the optical fiber 22A (step ST6b). As described above, the optical signal sequentially propagated from the optical sensor chip 21₁ to the optical sensor chip 21_{Z} is reflected at the optical sensor chip 21_{Z}, and is sequentially propagated from the optical sensor chip 21_{Z} to the optical sensor chip 21₁.

Also in the propagation path of the optical signal from the optical sensor chip 21_{Z} to the optical sensor chip 21₁, the processing similar to that in steps ST1b to ST6b illustrated in FIG. 7 is performed. In this case, in step ST1b, the spot-size converter unit 2103 converts the spot size of the optical signal output from the optical sensor chip at the preceding stage on the basis of the waveguide 2104B. Furthermore, in step ST5b, the spot-size converter unit 2101 converts the spot size of the optical signal emitted from the changer unit 2102A on the basis of the optical fiber 22B.

In step ST4b, the signal component with the resonance wavelength λₖ set in the optical sensor chip 21ₖ circulates in the waveguide 2106 and resonates, and the intensity thereof decreases in proportion to the moisture content of the specimen 100. In addition, the signal component with the wavelength λ (≠ λₖ) does not propagate through the waveguide 2106 but directly propagates through the waveguide 2104B and is emitted from the changer unit 2102A. That is, in the changer unit 2102A, the waveguide 2104B is provided close to the waveguide 2106, similarly to the waveguide 2104A illustrated in FIG. 6.

In the process of propagating the optical signal from the optical sensor chip 21₁ toward the optical sensor chip 21_{Z}, the characteristics of the optical signal change depending on the moisture content inside or around the specimen 100. Therefore, the optical signal propagating from the optical sensor chip 21_{Z} toward the optical sensor chip 21₁ may directly pass through the changer unit 2102A without propagating through the waveguide 2106.

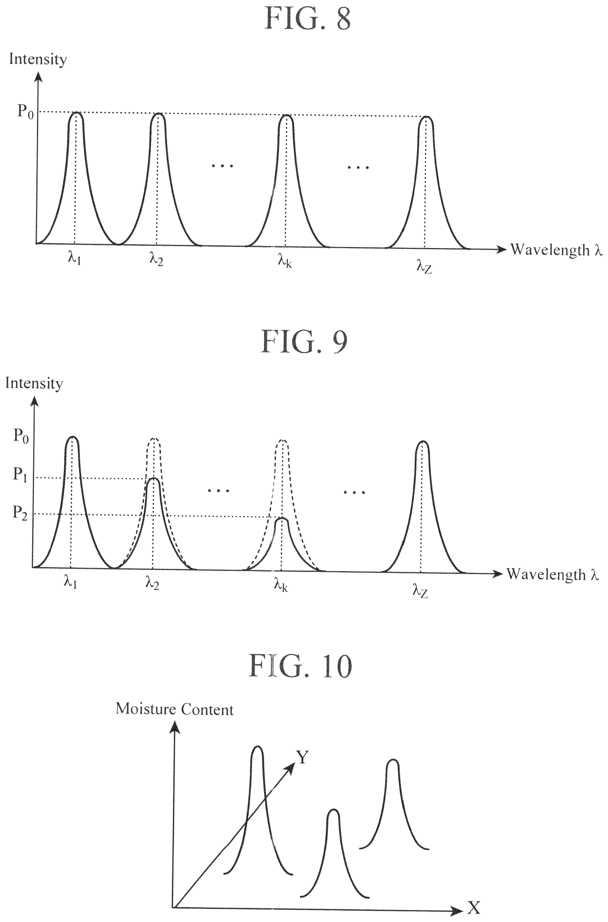

FIG. 8 is a graph illustrating a relationship between the wavelength and the intensity of an optical signal propagating through the changer unit 2102 included in the optical sensor chip 21ₖ of the optical sensor sheet 2 on which the specimen 100 is not disposed. In FIG. 8, the horizontal axis represents the wavelength of the optical signal, and the vertical axis represents the intensity of the optical signal. Since the specimen 100 is not disposed and there is no absorption of water present inside or around the specimen 100, the intensities of Z optical signals with the wavelengths λ₁ to λ_{Z} in the absorption wavelength band of water are constant at an intensity P₀.

FIG. 9 is a graph illustrating a relationship between the wavelength and the intensity of an optical signal propagating through the changer unit 2102 included in the optical sensor chip 21ₖ of the optical sensor sheet 2 on which the specimen 100 is disposed. In FIG. 9, the horizontal axis represents the wavelength of the optical signal, and the vertical axis represents the intensity of the optical signal. When the specimen 100 is disposed on the optical sensor sheet 2, the signal component with the wavelength λₖ is absorbed by moisture present inside or around the specimen 100.

For example, the signal component with the wavelength λ₂ has an intensity P₁ lower than the intensity P₀ when the specimen 100 is not disposed, and the signal component with the wavelength λₖ has an intensity P₂ lower than the intensity P₀. This means that, among the optical signals reciprocated from the optical sensor chip 21₁ to the optical sensor chip 21_{Z}, the signal component with the resonance wavelength λ₂ set in the optical sensor chip 21₂ is absorbed by moisture present inside or around the specimen 100, and the signal component with the resonance wavelength λₖ set in the optical sensor chip 21ₖ is absorbed by moisture present inside or around the specimen 100.

The received signal analyzer unit 4 can measure the moisture content inside or around the specimen 100 by analyzing the amount of change (P₀-P₁ and P₀-P₂) in the intensity of the signal component with the resonance wavelength λₖ set in each of the Z optical sensor chips 21ₖ.

FIG. 10 is a graph illustrating a relationship between the position of the optical sensor chip 21ₖ of the optical sensor sheet 2 on which the specimen 100 is disposed and the moisture content of the specimen 100. The received signal analyzer unit 4 calculates the reciprocating propagation time of a pulse using the pulse of an optical signal transmitted to the optical sensor chip 21₁ and the pulse of an optical signal received from the optical sensor chip 21₁, and specifies the position of the optical sensor chip 21ₖ in the XY coordinate system on the optical sensor sheet 2 on the basis of the reciprocating propagation time.

The received signal analyzer unit 4 measures the moisture content of the specimen 100 at each position of the optical sensor chip 21ₖ, and outputs information indicating the moisture content at each position of the optical sensor chip 21ₖ to the display unit 5 as measurement results. For example, as illustrated in FIG. 10, the display unit 5 displays a three-dimensional graph illustrating the moisture content of the specimen 100 at each position of the optical sensor chip 21ₖ in the XY coordinate system on the optical sensor sheet 2.

Note that, in the above description, the case of detecting the moisture content of the specimen 100 at each position of the optical sensor chip 21ₖ has been described, but the Z optical sensor chips 21ₖ may change the characteristics of the input optical signal depending on a plurality of types of states of the specimen 100. For example, the Z optical sensor chips 21ₖ may include an optical sensor chip that changes the characteristics of the optical signal depending on the temperature of the specimen 100, an optical sensor chip that changes the characteristics of the optical signal depending on the moisture content of the specimen 100, and an optical sensor chip that changes the characteristics of the optical signal depending on the pressure from the specimen 100.

In addition, a part of the Z optical sensor chips 21ₖ may be optical sensor chips that change the characteristics of the optical signal depending on the moisture content of the specimen 100, and the remaining may be optical sensor chips that change the characteristics of the optical signal depending on the temperature of the specimen 100.

Furthermore, the changer unit 2102A may include a ring resonator that changes the characteristics of the optical signal depending on the moisture content of the specimen 100 and a ring resonator that changes the characteristics of the optical signal depending on the temperature of the specimen 100. As a result, the characteristics of the optical signal input to one optical sensor chip can be changed depending on a plurality of types of states of the specimen 100.

Moreover, the optical sensor sheet 2 may be a sheet in which a plurality of units including a plurality of optical sensor chips that change the characteristics of the optical signal depending on each of a plurality of types of states of the specimen 100 are continuously connected. For example, in the above unit, an optical sensor chip that detects the temperature of the specimen 100, an optical sensor chip that detects the moisture content of the specimen 100, and an optical sensor chip that detects the pressure from the specimen 100 are optically connected via the optical fiber 22, and a plurality of such units are continuously connected in the optical sensor sheet 2.

In the case of detecting the temperature of the specimen 100, for example, the waveguide 2106 in which the effective curvature radius R changes with a temperature change ΔT is used in the changer unit 2102A. In this case, the waveguide 2106 satisfying 2π × ΔR × n_{eff} = m × Δλ, which is a relational expression indicating the correspondence relationship between the amount of change ΔR in curvature radius and the amount of shift Δλ in resonance wavelength, is used. The received signal analyzer unit 4 calculates the amount of change ΔR in the curvature radius of the waveguide 2106 on the basis of the amount of shift Δλ of the resonance wavelength between a transmitted signal and a received signal, and calculates the temperature change ΔT using ΔR.

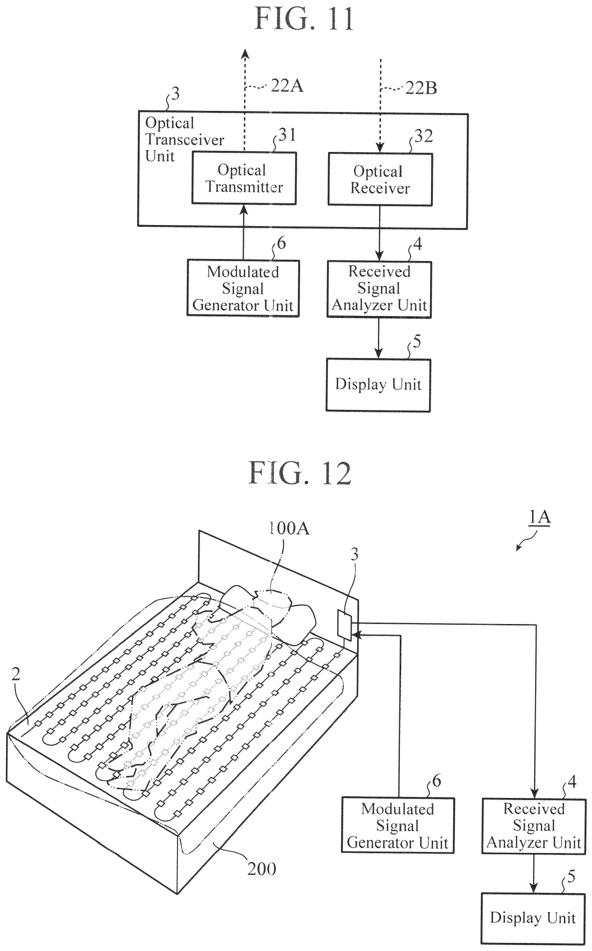

FIG. 11 is a block diagram illustrating a first configuration example of a measurement device, in which a modulated signal generator unit 6 is added to the measurement device illustrated in FIG. 1. The modulated signal generator unit 6 generates a modulated signal used for measuring the state of the specimen 100. For example, the modulated signal is a phase modulated signal or a frequency modulated signal. The optical transmitter 31 included in the optical transceiver unit 3 transmits, to the optical sensor chip 21₁, an optical signal modulated using the modulated signal generated by the modulated signal generator unit 6 as a carrier wave.

For example, the modulated signal generator unit 6 outputs a phase-modulated pulse signal to the optical transmitter 31 on the basis of the time of flight (ToF) LiDAR system. As a result, the pulse signal reciprocates between the optical sensor chip 21₁ and the optical sensor chip 21_{Z}. The received signal analyzer unit 4 calculates a correlation value between the pulse of the received signal received from the optical sensor chip 21₁ by the optical receiver 32 and the pulse of the transmitted signal, and measures the state of the specimen 100 on the basis of the calculated correlation value. The measurement results by the received signal analyzer unit 4 are displayed on the display unit 5.

FIG. 12 is a configuration diagram illustrating a measurement system 1A that is a first modification of the measurement system 1. As illustrated in FIG. 12, the optical sensor sheet 2 is laid on a bed 200, and the specimen is a person 100A lying on the bed 200. By the person 100A lying on the bed 200, the person 100A is disposed on the optical sensor sheet 2. The measurement system 1A measures the state of the person 100A lying on the bed 200.

The received signal analyzer unit 4 can measure the temperature, the amount of perspiration, or the sleeping position of the person 100A on the basis of the change in the characteristics of the optical signal for each optical sensor chip 21ₖ of the optical sensor sheet 2. For example, the received signal analyzer unit 4 measures the amount of perspiration of the person 100A and the temporal change thereof at each position of the optical sensor chip 21ₖ, and outputs the measurement results to the display unit 5. As a result, the display unit 5 can graphically display the temporal change in the distribution of the amount of perspiration of the person 100A.

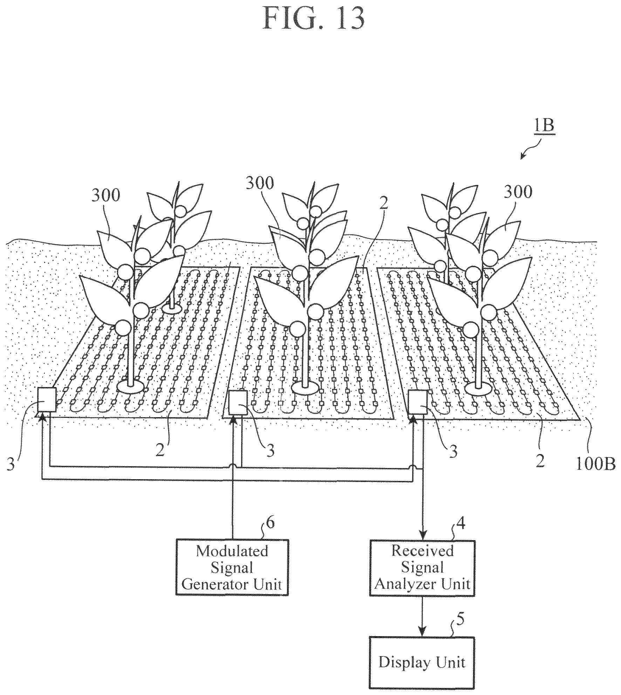

Furthermore, FIG. 13 is a configuration diagram illustrating a measurement system 1B that is a second modification of the measurement system 1. As illustrated in FIG. 13, the optical sensor sheet 2 is buried in a farmland, and the specimen is farmland soil 100B. The measurement system 1B measures the state of the farmland soil. For example, the received signal analyzer unit 4 measures the temperature, the moisture content, or the nutrient component of the soil 100B on the basis of the change in the characteristics of an optical signal for each optical sensor chip 21ₖ of the optical sensor sheet 2.

In addition, in the case of detecting the carbon dioxide concentration or the calcium concentration of the soil 100B, the wavelength λₖ in the absorption wavelength band of carbon dioxide or calcium is set in the changer unit 2102A included in each of Z optical sensor chips 21ₖ. Similarly to the measurement of the moisture content of the specimen 100, the intensity of the optical signal circulated in a ring resonator and resonated decreases depending on the carbon dioxide concentration or the calcium concentration of the soil 100B. The received signal analyzer unit 4 measures the temperature, the moisture content, or the nutrient component of the soil 100B and the temporal change thereof at each position of the optical sensor chip 21ₖ, on the basis of the temporal change in the intensity of the optical signal, and outputs the measurement results to the display unit 5. As a result, the display unit 5 can graphically display the temporal change in the distribution of the temperature, the moisture content, or the nutrient component of the soil 100B.

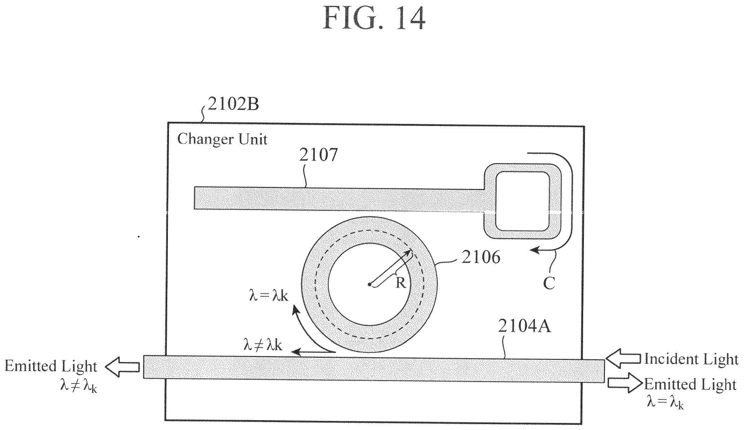

FIG. 14 is a configuration diagram illustrating a changer unit 2102B that is a second modification of the changer unit 2102. In FIG. 14, the changer unit 2102B is obtained by adding a waveguide 2107 to the changer unit 2102A. The waveguide 2107 includes a band-like line path provided close to the waveguide 2106 which is a ring resonator and a ring-like line path provided at an end thereof.

The optical signal component with the resonance wavelength λₖ propagates from the waveguide 2104A to the waveguide 2106 and resonates, then propagates through the waveguide 2107, and the optical path is reflected in a direction indicated by an arrow C. As illustrated in FIG. 14, the reflected optical signal component propagates through the waveguide 2106 again and resonates, and then propagates through the waveguide 2104A toward the optical transceiver unit 3 as the emitted light with the wavelength λₖ.

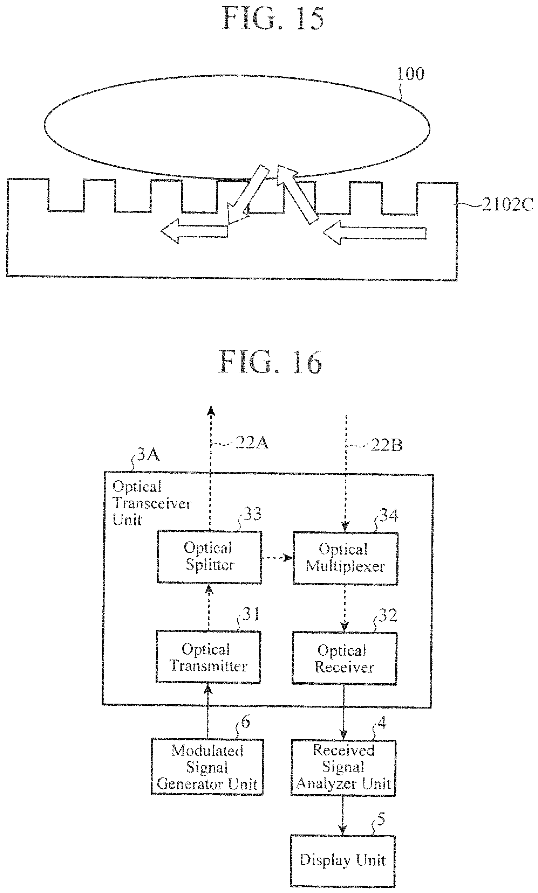

FIG. 15 is a configuration diagram illustrating a changer unit 2102C that is a third modification of the changer unit 2102. In FIG. 15, the changer unit 2102B is an optical element that outputs a part of the optical signal propagating through the waveguide 2104A to the outside, and receives reflected light obtained by reflecting the light output to the outside. For example, the changer unit 2102B is implemented by a grating coupler.

The characteristics of the optical signal emitted from the changer unit 2102B are changed depending on the state of the specimen 100 outside, and the optical signal is reflected by the specimen 100, and input to the changer unit 2102B again. The optical signal whose characteristics have been changed depending on the state of the specimen 100 propagates through the waveguide 2104A and is output to the spot-size converter unit 2103.

FIG. 16 is a block diagram illustrating a second configuration example of the measurement device. In FIG. 16, the measurement device includes an optical transceiver unit 3A, the received signal analyzer unit 4, the display unit 5, and the modulated signal generator unit 6. The optical transceiver unit 3A includes the optical transmitter 31, the optical receiver 32, an optical splitter 33, and an optical multiplexer 34.

For example, the modulated signal generator unit 6 outputs a frequency modulated signal such as a chirped sine wave to the optical transmitter 31 included in the optical transceiver unit 3A on the basis of the frequency-modulated-continuous-wave (FMCW) LiDAR system. The optical transmitter 31 outputs a modulated optical signal to the optical splitter 33 using the frequency modulated signal generated by the modulated signal generator unit 6 as a carrier wave.

The optical splitter 33 splits the optical signal generated by the optical transmitter 31 to the optical sensor chip 21₁ and the optical multiplexer 34. The optical transmitter 31 transmits the optical signal split by the optical splitter 33 to the optical sensor chip 21₁ via the optical fiber 22A. As a result, the optical signal reciprocates between the optical sensor chip 21₁ and the optical sensor chip 21_{Z}.

The optical multiplexer 34 multiplexes the optical signal received from the optical sensor chip 21₁ via the optical fiber 22B and the transmitting optical signal split by the optical splitter 33, and outputs the multiplexed optical signal to the optical receiver 32. The received signal analyzer unit 4 performs Fourier analysis on the received signal received from the optical sensor chip 21₁ by the optical receiver 32, thereby calculating the amount of change in amplitude and the amount of frequency shift between the transmitted signal and the received signal, and measures the state of the specimen 100 on the basis of the calculated amount of change in amplitude and amount of frequency shift. The measurement results are displayed on the display unit 5.

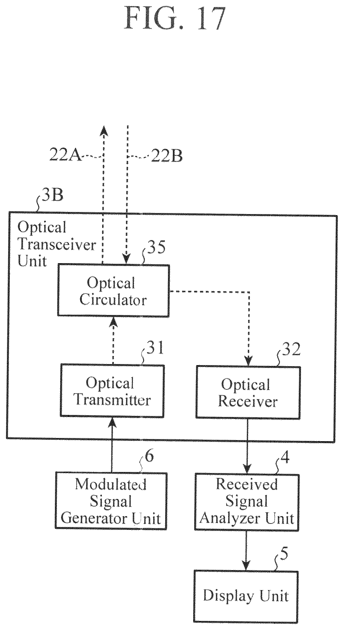

FIG. 17 is a block diagram illustrating a third configuration example of the measurement device. In FIG. 17, the measurement device includes an optical transceiver unit 3B, the received signal analyzer unit 4, the display unit 5, and the modulated signal generator unit 6. The optical transceiver unit 3B includes the optical transmitter 31, the optical receiver 32, and an optical circulator 35. The optical circulator 35 switches the output destination of the optical signal input from the optical transmitter 31 to the optical fiber 22A, and switches the output destination of the optical signal from the optical fiber 22B to the optical receiver 32. As a result, the optical fiber 22A and the optical fiber 22B could be shared by one optical fiber in an example not being part of the invention.

In addition, the ring resonator illustrated in FIGS. 6 and 14 may be replaced with an optical element having a phase shifter function or a frequency shifter function, or the ring resonator may have the phase shifter function or the frequency shifter function. For example, a member formed of a material different from that of the waveguide 2106 is stacked on the waveguide 2106 which is a ring resonator. The changer unit 2102A changes the characteristics of the optical signal depending on the material of the member stacked on the waveguide 2106.

For example, in a case where a magnetic body is stacked on the waveguide 2106, the phase of the optical signal circulated in the waveguide 2106 changes in proportion to the intensity of the magnetic field generated around the waveguide 2106. The waveguide effective refractive index n_{eff} of the waveguide 2106 also changes depending on the change in the phase of the optical signal. The received signal analyzer unit 4 calculates the amount of shift Δλ of the resonance wavelength between the transmitted signal and the received signal using the relationship of 2π × R × Δn_{eff} = m × Δλ in the waveguide 2106. The received signal analyzer unit 4 calculates the amount of change Δn_{eff} in the waveguide effective refractive index of the waveguide 2106 using the calculated amount of shift Δλ in resonance wavelength, and calculates the intensity of the magnetic field using the calculated Δn_{eff}. As a result, the optical sensor chip 21ₖ can detect the magnetic field from the specimen 100.

Furthermore, the waveguide 2106 may be a waveguide on which a member that changes the waveguide effective refractive index n_{eff} by binding to DNA such as graphene is stacked. When DNA is present around the waveguide 2106, the phase of the optical signal circulated in the waveguide 2106 changes. The waveguide effective refractive index n_{eff} of the waveguide 2106 also changes depending on the change in the phase of the optical signal. Similarly to the case of detecting the magnetic field, the received signal analyzer unit 4 calculates the amount of shift Δλ in resonance wavelength between the transmitted signal and the received signal using the relationship of 2π × R × Δn_{eff} = m × Δλ in the waveguide 2106. The received signal analyzer unit 4 calculates the amount of change Δn_{eff} in the waveguide effective refractive index of the waveguide 2106 using the amount of shift Δλ in resonance wavelength, and calculates the binding amount of DNA using the calculated Δn_{eff}. As a result, the optical sensor chip 21ₖ can detect the DNA of the specimen 100.

The functions of the optical transceiver unit 3, the received signal analyzer unit 4, the display unit 5, and the modulated signal generator unit 6 in the measurement device illustrated in FIG. 11 are implemented by a processing circuitry. That is, the measurement device includes a processing circuitry for performing the processing from step ST1 to step ST3 illustrated in FIG. 4. The processing circuitry may be dedicated hardware, or may be a central processing unit (CPU) that executes a program stored in a memory.

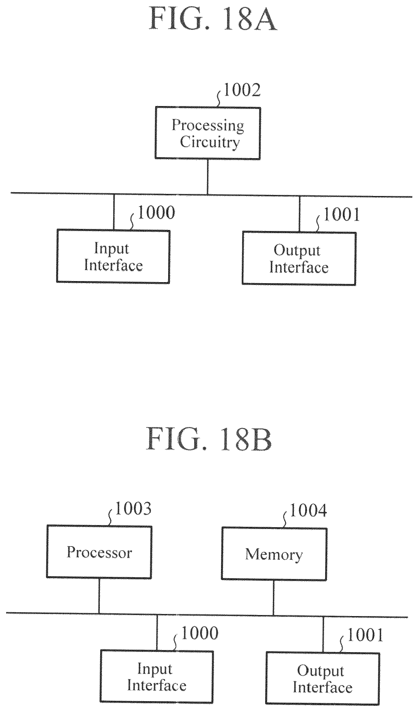

FIG. 18A is a block diagram illustrating a hardware configuration that implements the functions of the measurement device illustrated in FIG. 11. FIG. 18B is a block diagram illustrating a hardware configuration that executes software that implements the functions of the measurement device illustrated in FIG. 11. In FIGS. 18A and 18B, an input interface 1000 is an interface that relays an electrical signal corresponding to a signal from the optical sensor sheet 2. An output interface 1001 is an interface that relays measurement result information output to the display.

In a case where the processing circuitry is a processing circuitry 1002 of dedicated hardware illustrated in FIG. 18A, the processing circuitry 1002 corresponds to, for example, a single circuit, a composite circuit, a programmed processor, a parallel programmed processor, an application specific integrated circuit (ASIC), a field-programmable gate array (FPGA), or a combination thereof. The functions of the optical transceiver unit 3, the received signal analyzer unit 4, the display unit 5, and the modulated signal generator unit 6 in the measurement device may be implemented by separate processing circuitries, or these functions may be collectively implemented by one processing circuitry.

In a case where the processing circuitry is a processor 1003 illustrated in FIG. 18B, the functions of the optical transceiver unit 3, the received signal analyzer unit 4, the display unit 5, and the modulated signal generator unit 6 are implemented by software, firmware, or a combination of software and firmware. Note that the software or firmware is described as a program and stored in a memory 1004.

The processor 1003 reads and executes the program stored in the memory 1004, thereby implementing the functions of the optical transceiver unit 3, the received signal analyzer unit 4, the display unit 5, and the modulated signal generator unit 6 in the measurement device. For example, the measurement device includes the memory 1004 for storing a program that allows the processing from step ST1 to step ST3 in the flowchart illustrated in FIG. 4 to be performed when executed by the processor 1003. These programs cause a computer to execute procedures or methods of processing performed by the optical transceiver unit 3, the received signal analyzer unit 4, the display unit 5, and the modulated signal generator unit 6. The memory 1004 may be a computer-readable storage medium that stores a program for causing a computer to function as the optical transceiver unit 3, the received signal analyzer unit 4, the display unit 5, and the modulated signal generator unit 6.

The memory 1004 corresponds, for example, to a nonvolatile or volatile semiconductor memory such as a random access memory (RAM), a read only memory (ROM), a flash memory, an erasable programmable read only memory (EPROM), or an electrically-EPROM (EEPROM), a magnetic disk, a flexible disk, an optical disk, a compact disk, a mini disk, a DVD, or the like.

Some of the functions of the optical transceiver unit 3, the received signal analyzer unit 4, the display unit 5, and the modulated signal generator unit 6 in the measurement device may be implemented by dedicated hardware, and some of the functions may be implemented by software or firmware. For example, the functions of the optical transceiver unit 3 and the received signal analyzer unit 4 are implemented by the processing circuitry 1002 that is dedicated hardware, and the functions of the display unit 5 and the modulated signal generator unit 6 are implemented by the processor 1003 reading and executing the program stored in the memory 1004. As described above, the processing circuitry can implement the functions by hardware, software, firmware, or a combination thereof.

As described above, the optical sensor sheet 2 according to the first embodiment includes Z optical sensor chips 21ₖ that change the characteristics of an input optical signal depending on the state of the specimen 100 and output the optical signal with the changed characteristics, the optical fiber 22 that propagates the optical signal input to and output from the Z optical sensor chips 21ₖ, and the sheet member 23 including the Z optical sensor chips 21ₖ.

Since the optical signal is input and output between the optical sensor chip 21ₖ and the optical sensor chip 21ₖ₋₁ through the optical fiber 22, the optical sensor device according to the first embodiment can detect the state of the specimen without using an electric signal for a specimen-state detection signal and without providing a sensor circuit unit for each sensor chip. As a result, power consumption is reduced as compared with the sensor described in Patent Literature 1.

Furthermore, in the optical sensor sheet 2, since the Z optical sensor chips 21ₖ are arranged on the sheet member 23, sensing can be performed from various perspectives on the basis of the shape of an inspection target (= the specimen 100).

In addition, the Z optical sensor chips 21ₖ are provided on the sheet member 23, and change the characteristics of the input optical signal depending on the state of the specimen 100 disposed on the sheet member 23 or the specimen 100 disposed around the sheet member 23. Even in the optical sensor sheet 2 configured as described above, it is possible to detect the state of the specimen without using an electric signal for a specimen-state detection signal and without providing a sensor circuit unit for each sensor chip. Note that, by meandering the Z optical sensor chips 21ₖ connected via the optical fiber 22, the optical sensor chips 21ₖ can be provided on the sheet member 23 with various shapes, and the degree of freedom in the arrangement of the optical sensor chips 21ₖ is high, and the arrangement density is improved. As a result, the optical sensor sheet 2 can be disposed even in a narrow space in which the optical sensor sheet is conventionally difficult to be disposed.

In the optical sensor sheet 2 according to the first embodiment, the Z optical sensor chips 21ₖ change the characteristics of the input optical signal depending on the state of the specimen 100 disposed on the sheet member 23 or disposed around the sheet member 23. Since the state of the specimen 100 can be detected at each position of the optical sensor chip 21ₖ, the measurement range of the state of the specimen 100 is extended.

In the optical sensor sheet 2 according to the first embodiment, the Z optical sensor chips 21ₖ change the characteristics of the input optical signal depending on a plurality of types of states of the specimen 100. Since the plurality of types of states of the specimen 100 can be detected for each optical sensor chip 21ₖ, the measurement range of the state of the specimen 100 is extended.

In the optical sensor sheet 2 according to the first embodiment, a member formed of a material different from that of the waveguide 2106 is stacked on the waveguide 2106. The changer unit 2102A changes the characteristics of the optical signal depending on the material of the member stacked on the waveguide 2106. Since the plurality of types of states of the specimen 100 can be detected for each optical sensor chip 21ₖ, the measurement range of the state of the specimen 100 is extended.

The measurement system 1 according to the first embodiment includes the optical sensor sheet 2, the optical transceiver unit 3 that transmits an optical signal to the optical sensor chip 21ₖ in the optical sensor sheet 2 and receives an optical signal propagated through the Z optical sensor chips 21ₖ provided continuously in the optical sensor sheet 2, and the received signal analyzer unit 4 that measures the state of the specimen 100 by analyzing a signal received by the optical transceiver unit 3. Since the received signal analyzer unit 4 measures the state of the specimen 100 for each optical sensor chip 21ₖ, the measurement range of the state of the specimen 100 is extended.

The measurement system 1 according to the first embodiment includes the modulated signal generator unit 6 that generates a modulated signal used for measuring the state of the specimen 100. The optical transceiver unit 3 modulates an optical signal using the modulated signal generated by the modulated signal generator unit 6, and transmits the modulated optical signal to the optical sensor chip 21₁. As a result, it is possible to reciprocate the optical signal between the optical sensor chip 21₁ and the optical sensor chip 21_{Z} with high quality.

### Second Embodiment

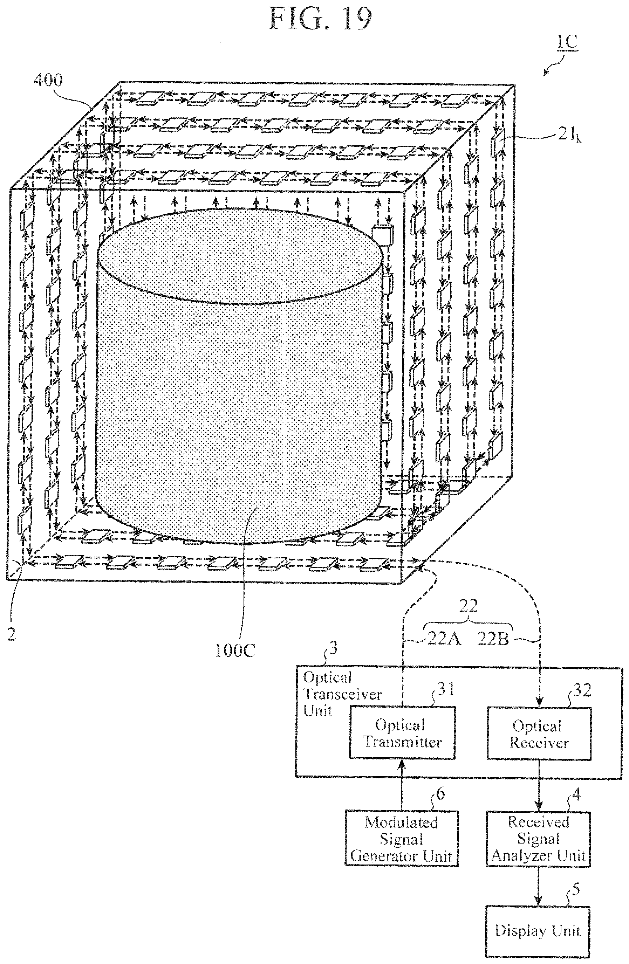

FIG. 19 is a configuration diagram illustrating a measurement system 1C according to a second embodiment. While the measurement systems 1, 1A, and 1B according to the first embodiment use the optical sensor sheet 2 disposed two-dimensionally, the measurement system 1C uses the optical sensor sheet 2 disposed three-dimensionally in a closed space. For example, by providing the optical sensor sheet 2 in such a manner as to cover a columnar specimen 100C, the measurement system 1C can determine the entire specimen 100C as a measurement portion, and the measurement range is extended.

In FIG. 19, since the optical sensor sheet 2 is provided in such a manner as to cover the columnar specimen 100C, the optical sensor sheet 2 is attached to all the inner wall surfaces of a box 400. By disposing the specimen 100C inside the box 400, the optical sensor sheet 2 can detect the state of the specimen 100C. For example, in a case where the optical sensor sheet 2 is two-dimensionally disposed, a state cannot be detected at a portion of the specimen 100C on the side where the optical sensor chip 21ₖ is not close to the specimen, but the entire specimen 100C can be determined as a measurement portion by three-dimensionally disposing the optical sensor sheet 2 in the closed space as illustrated in FIG. 19.

Note that the box 400 does not need to be a closed space. For example, the optical sensor sheet 2 may be three-dimensionally disposed in a space where a part is opened, such as a pipe or a tunnel. In this case, the optical sensor sheet 2 cannot be disposed in the opened portion, but the optical sensor sheet 2 can be disposed around the specimen 100C except for this portion. Therefore, the optical sensor system 1C can determine all the portions of the specimen 100C facing the optical sensor sheet 2 as measurement portions.

In addition, it is assumed that the closed space is a cardboard box including the optical sensor sheet 2 on the inner wall surface, a food is disposed as the specimen 100C inside the cardboard box, and the optical sensor chip 21ₖ is, for example, an optical sensor chip that detects the moisture content of the food or the humidity inside the cardboard box. In this case, the measurement system 1C can measure the moisture distribution of the food or the humidity distribution of the food inside the cardboard box using the optical sensor sheet 2. A food manager can recognize the temporal change in the moisture distribution of the food or the humidity distribution of the food on the basis of the measurement results of the moisture distribution of the food or the humidity distribution of the food. The measurement results can also be used to predict the occurrence of rotting of the food.

Furthermore, the closed space may be a bathroom including the optical sensor sheet 2 on the wall surface, and the atmosphere inside the bathroom may be the specimen 100C. The optical sensor chip 21ₖ provided inside the bathroom is, for example, an optical sensor chip that detects the humidity of the atmosphere inside the bathroom. The measurement system 1C measures the humidity distribution of the atmosphere inside the bathroom using the optical sensor sheet 2. As a result, the temporal change in the humidity distribution of the atmosphere inside the bathroom can be recognized. In addition, the measurement results of the humidity distribution of the atmosphere inside the bathroom can also be used to predict the occurrence of mold on the bathroom wall surface.

As described above, in the measurement system 1C according to the second embodiment, the optical sensor sheet 2 is provided in such a manner as to cover the specimen 100C. As a result, the measurement system 1C can determine the entire specimen 100C as a measurement portion, and the measurement range is extended.

### Third Embodiment

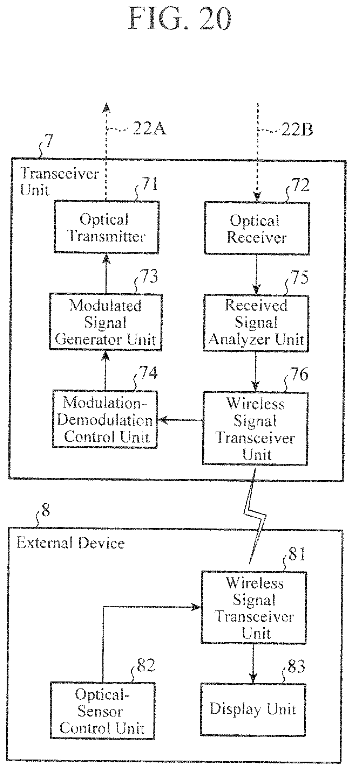

FIG. 20 is a block diagram illustrating a configuration of a measurement device according to a third embodiment. In FIG. 20, the measurement device according to the third embodiment includes a transceiver unit 7 and an external device 8. The transceiver unit 7 and the external device 8 are configured to be capable of transmitting and receiving wireless signals. The transceiver unit 7 includes an optical transmitter 71, an optical receiver 72, a modulated signal generator unit 73, a modulation-demodulation control unit 74, a received signal analyzer unit 75, and a wireless signal transceiver unit 76. For example, the transceiver unit 7 is an IoT sensor. The external device 8 includes a wireless signal transceiver unit 81, an optical-sensor control unit 82, and a display unit 83. For example, the external device 8 is implemented by a mobile phone terminal, a smartphone, a personal digital assistant (PDA), or a personal computer (PC).

In the transceiver unit 7, the optical transmitter 71 transmits an optical signal to the optical sensor chip 21₁ through the optical fiber 22A. The optical receiver 72 receives an optical signal from the optical sensor chip 21₁ through the optical fiber 22B. The optical transmitter 71 generates a transmitting optical signal using light emitted from a light emitting element as a light source and the modulated signal generated by the modulated signal generator unit 73, and outputs the generated optical signal to the optical fiber 22A.

The optical receiver 72 acquires an electrical signal converted from an optical signal by a light receiving element. The light emitting element and the light receiving element may be provided separately, or may be an optical sensor in which the light emitting element and the light receiving element are integrated into one. The optical transceiver unit including the optical transmitter 71 and the optical receiver 72 may be provided in an external device provided separately from the optical sensor sheet 2, or may be provided in the optical sensor sheet 2. For example, the optical transceiver unit may be integrated on an InP substrate provided on the optical sensor sheet 2.

The modulated signal generator unit 73 may generate a modulated signal to be used to measure a state designated by a control signal among a plurality of types of states of the specimen 100, and output the generated modulated signal to the optical transmitter 71. The optical transmitter 71 generates an optical signal to which modulation corresponding to the measurement of the state designated by the control signal has been applied. As a result, it is possible to measure the state of the specimen 100 using the optical signal to which modulation corresponding to the measurement of the designated state has been applied. For example, the modulated signal generator unit 73 generates a phase modulated signal in a case where the moisture content of the specimen 100 is designated by the control signal, and generates a frequency modulated signal in a case where the temperature of the specimen 100 is designated by the control signal. As a result, it is possible to generate an optical signal that has been phase-modulated to measure the moisture content of the specimen 100 or frequency-modulated to measure the temperature of the specimen 100.

The modulation-demodulation control unit 74 demodulates the control signal received from the external device 8 by the wireless signal transceiver unit 76, and outputs the demodulated control signal to the modulated signal generator unit 73. For example, the modulation-demodulation control unit 74 compensates for the degradation of the signal quality of the control signal wirelessly transmitted from the external device 8, and outputs the control signal in which the degradation of the signal quality has been compensated to the modulated signal generator unit 73. For example, the modulation-demodulation control unit 74 compensates for the degradation of the signal quality by performing error detection of a signal being wirelessly transmitted and corrects the detected error.

The received signal analyzer unit 75 measures the state of the specimen 100 by analyzing the optical signal received from the optical sensor chip 21₁ by the optical receiver 72. Note that the optical sensor sheet 2 may be two-dimensionally disposed as described in the first embodiment, or may be three-dimensionally disposed as described in the second embodiment.

The wireless signal transceiver unit 76 is a wireless communication unit for transmitting and receiving a wireless signal to and from the external device 8, and is implemented by, for example, a communication device that performs near field communication represented by Bluetooth (registered trademark) or a WiFi router. The wireless signal transceiver unit 76 transmits measurement result information measured by the received signal analyzer unit 75 to the external device 8.

In the external device 8, the wireless signal transceiver unit 81 is a wireless communication unit for transmitting and receiving a wireless signal to and from the transceiver unit 7, and is implemented by, for example, a communication device that performs near field communication represented by Bluetooth (registered trademark) or a WiFi router. The wireless signal transceiver unit 81 transmits a control signal from the optical-sensor control unit 82 to the transceiver unit 7, and receives measurement result information from the transceiver unit 7.

The optical-sensor control unit 82 is a control unit that generates a control signal for designating the state of the specimen 100. The wireless signal transceiver unit 81 transmits the control signal generated by the optical-sensor control unit 82 to the wireless signal transceiver unit 76 by wireless communication. The display unit 83 displays information on a display mounted on the external device 8. For example, the display unit 83 causes the display to display the measurement result information received from the transceiver unit 7 by the wireless signal transceiver unit 81.

As described above, the measurement device according to the third embodiment includes the wireless signal transceiver unit 76 that performs wireless communication with the external device 8 including the optical-sensor control unit 82 and the display unit 83. The wireless signal transceiver unit 76 transmits information of the measurement results measured by the received signal analyzer unit 75 to the external device 8. The display unit 83 displays the measurement result information. The optical-sensor control unit 82 generates a control signal for designating the state of the specimen 100. The modulated signal generator unit 73 generates a modulated signal to be used to measure the state of the specimen 100 designated by the control signal, and outputs the generated modulated signal to the optical transmitter 71. For example, the user carrying the external device 8 can control the measurement of the state of the specimen 100 using the optical sensor sheet 2 and the transceiver unit 7 from a remote place. In addition, it is possible to designate any state of the specimen 100 on the basis of the measurement results displayed on the display unit 83.

Note that it is possible to combine the embodiments, modify any component of each embodiment, or omit any component of each embodiment.

### INDUSTRIAL APPLICABILITY

The optical sensor device according to the present disclosure can be used to measure the state of various specimens.

### REFERENCE SIGNS LIST

1, and 1A to 1C: Measurement system, 2: Optical sensor sheet, 3, 3A, and 3B: Optical transceiver unit, 4 and 75: Received signal analyzer unit, 5 and 83: Display unit, 6 and 73: Modulated signal generator unit, 7: Transceiver unit, 8: External device, 21₁, 21₂, 21ₖ, 21ₖ₋₁, 21_{Z-1}, and 21_{Z}: Optical sensor chip, 22, 22A, and 22B: Optical fiber, 23: Sheet member, 24: Adhesive layer, 31 and 71: Optical transmitter, 32 and 72: Optical receiver, 33: Optical splitter, 34: Optical multiplexer, 35: Optical circulator, 74: Modulation-demodulation control unit, 76 and 81: Wireless signal transceiver unit, 82: Optical-sensor control unit, 100 and 100C: Specimen, 100A: Person, 100B: Soil, 200: Bed, 400: Box, 1000: Input interface, 1001: Output interface, 1002: Processing circuitry, 1003: Processor, 1004: Memory, 2101 and 2103: Spot-size converter unit, 2102, and 2102A to 2102C: Changer unit, 2104A, 2104B, 2106, and 2107: Waveguide, 2105: Optical path folding unit

## Claims

1. An optical sensor device (2) comprising:
a plurality of optical sensor chips (21₁, 21₂, 21ₖ₋₁, 21ₖ, 21_{Z-1}, 21_{Z}) that have one or a plurality of changer units (2102, 2102A to 2102C) to change a characteristic of an input optical signal depending on a state of a specimen, and that output an optical signal with a changed characteristic;
a plurality of pairs of optical fibers (22A, 22B), through which an optical signal propagates, and via which the optical signal is input to and output from the plurality of optical sensor chips (21₁, 21₂, 21ₖ₋₁, 21ₖ, 21_{Z-1}, 21_{Z}), each pair connecting two adjacent optical sensor chips so that the plurality of pair of optical fibers (22, 22A, 22B) are connected in series by the plurality of optical sensor chips (21₁, 21₂, 21ₖ₋₁, 21ₖ, 21_{Z-1}, 21_{Z}); and
a chip holding member (23) to which the plurality of optical sensor chips (21₁, 21₂, 21ₖ₋₁, 21ₖ, 21_{Z-1}, 21_{Z}) are provided,
wherein each pair of optical fibers (22A, 22B) includes an optical fiber for transmission of an optical signal to the plurality of optical sensor chips, and an optical fiber for reception of an optical signal from the plurality of optical sensor chips.

2. The optical sensor device (2) according to claim 1, wherein
the chip holding member (23) is a sheet member whose shape is deformable.

3. The optical sensor device (2) according to claim 1, wherein
the plurality of optical sensor chips (21₁, 21₂, 21ₖ₋₁, 21ₖ, 21_{Z-1}, 21_{Z}) are continuous via the optical fibers (22, 22A, 22B).

4. The optical sensor device (2) according to claim 1, wherein
the plurality of optical sensor chips (21₁, 21₂, 21ₖ₋₁, 21ₖ, 21_{Z-1}, 21_{Z}) include: a first optical sensor chip (21₁) to input and output an optical signal to and from an optical transceiver unit (3, 3A, 3B); and a second optical sensor chip (21₂) to change a characteristic of an optical signal output from the optical sensor chip (21₂) at a preceding stage and to output an optical signal with a changed characteristic to the optical sensor chip (21₂) at a preceding stage by return, and
the first optical sensor chip (21₁) changes a characteristic of an optical signal from the optical transceiver unit (3, 3A, 3B), outputs an optical signal with a changed characteristic to the optical sensor chip at a subsequent stage, and outputs an optical signal having propagated through the plurality of optical sensor chips (21₁, 21₂, 21ₖ₋₁, 21ₖ, 21_{Z-1}, 21_{Z}) continuous between the first optical sensor chip (21₁) and the second optical sensor chip (21₂) to the optical transceiver unit (3, 3A, 3B).

5. The optical sensor device (2) according to claim 1, wherein
the optical sensor chip (21ₖ) includes:
a waveguide (2104A, 2104B) through which an optical signal propagates;
a spot-size converter unit (2101, 2103) to convert a spot size of an optical signal; and
one or a plurality of changer units (2102, 2102A to 2102C) to change at least one of an intensity characteristic, a phase characteristic, or a frequency characteristic of an optical signal depending on a state of the specimen (100, 100A to 100C).

6. The optical sensor device (2) according to claim 1, wherein
the plurality of optical sensor chips (21₁, 21₂, 21ₖ₋₁, 21ₖ, 21_{Z-1}, 21_{Z}) change a characteristic of an input optical signal depending on a plurality of types of states of the specimen (100, 100A to 100C).

7. The optical sensor device (2) according to claim 4, wherein
the second optical sensor chip (21_{Z}) includes a reflector unit (2105) to reflect an optical signal with a changed characteristic to the optical sensor chip at a preceding stage.

8. The optical sensor device (2) according to claim 5, wherein
the plurality of changer units (2102, 2102A to 2102B) change a characteristic of an input optical signal depending on different states of the specimen (100, 100A to 100C).

9. The optical sensor device (2) according to claim 5, wherein
the spot-size converter unit (2101, 2103) is a spot size converter or a grating coupler including a waveguide.

10. The optical sensor device (2) according to claim 5, wherein
each of the changer units (2102A) is a ring resonator (2106), a Mach-Zehnder interferometer, a combination of the ring resonator and the Mach-Zehnder interferometer, or an optical element to output a part of an optical signal propagating through the waveguide to outside and to input reflected light obtained by reflecting the light output to the outside.

11. The optical sensor device (2) according to claim 5, wherein
a member formed of a material different from a material of the waveguide (2106) is stacked on the waveguide (2106), and
each of the changer units (2102A) changes a characteristic of an input optical signal depending on a material of the member stacked on the waveguide (2106).

12. A measurement system (1, 1A to 1C) comprising:
the optical sensor device (2) according to any one of claims 1 to 11;
an optical transceiver unit (3, 3A, 3B) to transmit an optical signal to the optical sensor chip (21₁) in the optical sensor device (2) and to receive an optical signal propagated through the plurality of optical sensor chips (21₁, 21₂, 21ₖ₋₁, 21ₖ, 21_{Z-1}, 21_{Z}) provided in the optical sensor device (2); and
a received signal analyzer unit (4, 75) to measure a state of the specimen (100, 100A to 100C) by analyzing a signal received by the optical transceiver unit (3, 3A, 3B).

13. The measurement system (1, 1A to 1C) according to claim 12, further comprising a modulated signal generator unit (6) to generate a modulated signal used to measure a state of the specimen (100, 100A to 100C).

14. The measurement system (1, 1A to 1C) according to claim 12, wherein
the received signal analyzer unit (4) specifies each of positions of the plurality of optical sensor chips (21₁, 21₂, 21ₖ₋₁, 21ₖ, 21_{Z-1}, 21_{Z}) using a signal received by the optical transceiver unit (3, 3A, 3B) , and measures a state of the specimen (100, 100A to 100C) at each of the positions of the optical sensor chips (21₁, 21₂, 21ₖ₋₁, 21ₖ, 21_{Z-1}, 21_{Z}).

15. The measurement system (1, 1A to 1C) according to claim 13, further comprising a wireless communication unit (76) to perform wireless communication with an external device (8) including a display unit (83) and a control unit (82), wherein
the wireless communication unit (76) causes the display unit (83) to display measurement result information measured by the received signal analyzer unit (75) by transmitting the measurement result information to the external device (8),
the control unit (82) generates a control signal to specify a state of the specimen (100, 100A to 100C), and
the modulated signal generator unit (73) generates a modulated signal to be used to measure a state of the specimen (100, 100A to 100C) designated by the control signal, and outputs the modulated signal generated to the optical transceiver unit (71).

16. A measurement method to measure a state of the specimen (100, 100A to 100C) using the optical sensor device (2) according to any one of claims 1 to 11, the measurement method comprising:
transmitting an optical signal to the optical sensor chip in the optical sensor device (2) by an optical transceiver unit (3, 3A, 3B);
receiving an optical signal propagated through the plurality of optical sensor chips (21₁, 21₂, 21ₖ₋₁, 21ₖ, 21_{Z-1}, 21_{Z}) provided in the optical sensor device (2) by the optical transceiver unit (3, 3A, 3B); and
measuring a state of the specimen (100, 100A to 100C) by analyzing a signal received by the optical transceiver unit (3, 3A, 3B), by a received signal analyzer unit (4).

## Patentansprüche

1. Optische Sensoreinrichtung (2), umfassend:
eine Vielzahl von optischen Sensorchips (21₁, 21₂, 21ₖ₋₁, 21ₖ, 21_{z-1}, 21_{z}), die eine oder eine Vielzahl von Änderungseinheiten (2102, 2102A bis 2102C) aufweisen, um eine Charakteristik eines optischen Eingangssignals in Abhängigkeit von einem Zustand einer Probe zu ändern, und die ein optisches Signal mit einer geänderten Charakteristik ausgeben;
eine Vielzahl von Paaren optischer Fasern (22A, 22B), durch die ein optisches Signal läuft, und über die das optische Signal in die Vielzahl von optischen Sensorchips (21₁, 21₂, 21ₖ₋₁, 21ₖ, 21_{z-1}, 21_{z}) eingegeben und aus diesen ausgegeben wird, wobei jedes Paar zwei benachbarte optische Sensorchips verbindet, so dass die Vielzahl von Paaren optischer Fasern (22, 22A, 22B) durch die Vielzahl optischer Sensorchips (21₁, 21₂, 21ₖ₋₁, 21ₖ, 21_{z-1}, 21_{z}) in Reihe verbunden sind; und
ein Chiphalteelement (23), an dem die Vielzahl von optischen Sensorchips (21₁, 21₂, 21ₖ₋₁, 21ₖ, 21_{z-1}, 21_{z}) bereitgestellt sind,
wobei jedes Paar optischer Fasern (22A, 22B) eine optische Faser zur Übertragung eines optischen Signals an die Vielzahl von optischen Sensorchips und eine optische Faser zum Empfang eines optischen Signals von der Vielzahl von optischen Sensorchips umfasst.

2. Optische Sensoreinrichtung (2) nach Anspruch 1, wobei
das Chiphalteelement (23) ein flächiges Element ist, dessen Form verformbar ist.

3. Optische Sensoreinrichtung (2) nach Anspruch 1, wobei
die Vielzahl von optischen Sensorchips (21₁, 21₂, 21ₖ₋₁, 21ₖ, 21_{z-1}, 21_{z}) über die optischen Fasern (22, 22A, 22B) kontinuierlich sind.

4. Optische Sensoreinrichtung (2) nach Anspruch 1, wobei
die Vielzahl von optischen Sensorchips (21₁, 21₂, 21ₖ₋₁, 21ₖ, 21_{z-1}, 21_{z}) umfassen: einen ersten optischen Sensorchip (21₁) zum Eingeben und Ausgeben eines optischen Signals in und aus einer optischen Sende-/Empfangseinheit (3, 3A, 3B); und einen zweiten optischen Sensorchip (21₂) zum Ändern einer Charakteristik eines optischen Signals, das aus dem optischen Sensorchip (21₂) in einer vorangehenden Stufe ausgegeben wird, und zum sofortigen Ausgeben eines optischen Signals mit einer geänderten Charakteristik an den optischen Sensorchip (21₂) in einer vorangehenden Stufe, und
der erste optische Sensorchip (21₁) eine Charakteristik eines optischen Signals von der optischen Sende-/Empfangseinheit (3, 3A, 3B) ändert, ein optisches Signal mit einer geänderten Charakteristik an den optischen Sensorchip in einer nachfolgenden Stufe ausgibt, und ein optisches Signal, das durch die Vielzahl von optischen Sensorchips (21₁, 21₂, 21ₖ₋₁, 21ₖ, 21_{z-1}, 21_{z}) gelaufen ist, kontinuierlich zwischen dem ersten optischen Sensorchip (21₁) und dem zweiten optischen Sensorchip (21₂) an die optische Sende-/Empfangseinheit (3, 3A, 3B) ausgibt.

5. Optische Sensoreinrichtung (2) nach Anspruch 1, wobei
der optische Sensorchip (21ₖ) umfasst:
einen Wellenleiter (2104A, 2104B), durch den ein optisches Signal läuft;
eine Spotgrößen-Konvertereinheit (2101, 2103), um eine Spotgröße eines optischen Signals zu konvertieren; und
eine oder eine Vielzahl von Änderungseinheiten (2102, 2102A bis 2102C), um mindestens eine Intensitätscharakteristik, eine Phasencharakteristik oder eine Frequenzcharakteristik eines optischen Signals in Abhängigkeit von einem Zustand der Probe (100, 100A bis 100C) zu ändern.

6. Optische Sensoreinrichtung (2) nach Anspruch 1, wobei
die Vielzahl von optischen Sensorchips (21₁, 21₂, 21ₖ₋₁, 21ₖ, 21_{z-1}, 21_{z}) eine Charakteristik eines optischen Eingangssignals in Abhängigkeit von mehreren Typen von Zuständen der Probe (100, 100Abis 100C) ändern.

7. Optische Sensoreinrichtung (2) nach Anspruch 4,
wobei der zweite optische Sensorchip (21_{z}) eine Reflektoreinheit (2105) enthält, um ein optisches Signal mit einer veränderten Charakteristik zu dem optischen Sensorchip in einer vorhergehenden Stufe zu reflektieren.

8. Optische Sensoreinrichtung (2) nach Anspruch 5, wobei
die Vielzahl von Änderungseinheiten (2102, 2102 A bis 2102B) eine Charakteristik eines optischen Eingangssignals in Abhängigkeit von verschiedenen Zuständen der Probe (100, 100A bis 100C) ändern.

9. Optische Sensoreinrichtung (2) nach Anspruch 5, wobei
die Spotgrößen-Konvertereinheit (2101, 2103) ein Spotgrößen-Konverter oder ein Gitterkoppler mit einem Wellenleiter ist.

10. Optische Sensoreinrichtung (2) nach Anspruch 5, wobei
jede der Änderungseinheiten (2102A) ein Ringresonator (2106), ein Mach-Zehnder-Interferometer, eine Kombination aus dem Ringresonator und dem Mach-Zehnder-Interferometer oder ein optisches Element ist, um einen Teil eines durch den Wellenleiter laufenden optischen Signals nach außen auszugeben und reflektiertes Licht einzutragen, das durch Reflektieren des nach außen ausgegebenen Lichts erhalten wird.

11. Optische Sensoreinrichtung (2) nach Anspruch 5, wobei
ein Element, das aus einem Material gebildet ist, das sich von einem Material des Wellenleiters (2106) unterscheidet, auf den Wellenleiter (2106) aufgelegt wird, und
jede der Änderungseinheiten (2102A) eine Charakteristik eines optischen Eingangssignals in Abhängigkeit von einem Material des auf den Wellenleiter (2106) aufgelegten Elements ändert.

12. Messsystem (1, 1A bis 1C), umfassend:
die optische Sensoreinrichtung (2) nach einem der Ansprüche 1 bis 11;
eine optische Sende-/Empfangseinheit (3, 3A, 3B) zum Übertragen eines optischen Signals an den optischen Sensorchip (21₁) in der optischen Sensoreinrichtung (2) und zum Empfangen eines optischen Signals, das durch die Vielzahl von optischen Sensorchips (21₁, 21₂, 21ₖ₋₁, 21ₖ, 21_{z-1}, 21_{z}) läuft, die in der optischen Sensoreinrichtung (2) bereitgestellt sind; und
eine Empfangssignal-Analyseeinheit (4, 75) zum Messen eines Zustands der Probe (100, 100A bis 100C) durch Analysieren eines von der optischen Sende-/Empfangseinheit (3, 3A, 3B) empfangenen Signals.

13. Messsystem (1, 1A bis 1C) nach Anspruch 12, ferner umfassend eine Moduliertes-Signal-Erzeugungseinheit (6) zum Erzeugen eines modulierten Signals, das zur Messung eines Zustands der Probe (100, 100A bis 100C) genutzt wird.

14. Messsystem (1, 1A bis 1C) nach Anspruch 12,
wobei die Empfangssignal-Analyseeinheit (4) jede von Positionen der Vielzahl von optischen Sensorchips (21₁, 21₂, 21ₖ₋₁, 21ₖ, 21_{z-1}, 21_{z}) unter Verwendung eines von der optischen Sende-/Empfangseinheit (3, 3A, 3B) empfangenen Signals spezifiziert, und einen Zustand der Probe (100, 100Abis 100C) an jeder der Positionen der optischen Sensorchips (21₁, 21₂, 21ₖ₋₁, 21ₖ, 21_{z-1}, 21_{z}) misst.

15. Messsystem (1, 1A bis 1C) nach Anspruch 13, ferner umfassend eine drahtlose Kommunikationseinheit (76), um eine drahtlose Kommunikation mit einer externen Einrichtung (8) durchzuführen, die eine Anzeigeeinheit (83) und eine Steuereinheit (82) umfasst, wobei
die drahtlose Kommunikationseinheit (76) die Anzeigeeinheit (83) dazu veranlasst, von der Empfangssignal-Analyseeinheit (75) gemessene Messergebnisinformationen anzuzeigen, durch Übertragen der Messergebnisinformationen an die externe Einrichtung (8),
die Steuereinheit (82) ein Steuersignal erzeugt, um einen Zustand der Probe (100, 100A bis 100C) zu spezifizieren, und
die Moduliertes-Signal-Erzeugungseinheit (73) ein moduliertes Signal erzeugt, das zur Messung eines durch das Steuersignal bestimmten Zustands der Probe (100, 100A bis 100C) zu nutzen ist, und das erzeugte modulierte Signal an die optische Sende-/Empfangseinheit (71) ausgibt.

16. Messverfahren zum Messen eines Zustands der Probe (100, 100A bis 100C) unter Verwendung der optischen Sensoreinrichtung (2) nach einem der Ansprüche 1 bis 11, wobei das Messverfahren umfasst:
Übertragen eines optischen Signals an den optischen Sensorchip in der optischen Sensoreinrichtung (2) durch eine optische Sende-/Empfangseinheit (3, 3A, 3B);
Empfangen eines optischen Signals, das durch die Vielzahl von optischen Sensorchips (21₁, 21₂, 21ₖ₋₁, 21ₖ, 21_{z-1}, 21_{z}) läuft, die in der optischen Sensoreinrichtung (2) bereitgestellt sind, durch die optische Sende-/Empfangseinheit (3, 3A, 3B); und
Messen eines Zustands der Probe (100, 100A bis 100C) durch Analysieren eines von der optischen Sende-/Empfangseinheit (3, 3A, 3B) empfangenen Signals durch eine Empfangssignal-Analyseeinheit (4).

## Revendications

1. Dispositif capteur optique (2), comprenant :
une pluralité de puces de capteur optique (21₁, 21₂, 21ₖ₋₁, 21ₖ, 21_{Z-1}, 21_{Z}) qui présentent une ou une pluralité d'unités de changement (2102, 2102A à 2102C) pour changer une caractéristique d'un signal optique d'entrée en fonction d'un état d'un échantillon, et qui délivrent en sortie un signal optique avec une caractéristique changée ;
une pluralité de paires de fibres optiques (22A, 22B), à travers lesquelles un signal optique se propage, et à travers lesquelles le signal optique est entré vers et sorti de la pluralité de puces de capteur optique (21₁, 21₂, 21ₖ₋₁, 21ₖ, 21_{Z-1}, 21_{Z}), chaque paire connectant deux puces de capteur optique adjacentes de telle sorte que la pluralité de paires de fibres optiques (22, 22A, 22B) soient connectées en série par la pluralité de puces de capteur optique (21₁, 21₂, 21ₖ₋₁, 21ₖ, 21_{Z-1}, 21_{Z}) ; et
un élément de support de puces (23) sur lequel sont fournies la pluralité de puces de capteur optique (21₁, 21₂, 21ₖ₋₁, 21ₖ, 21_{Z-1}, 21_{Z}),
dans lequel chaque paire de fibres optiques (22A, 22B) inclut une fibre optique pour la transmission d'un signal optique à la pluralité de puces de capteur optique, et une fibre optique pour la réception d'un signal optique à partir de la pluralité de puces de capteur optique.

2. Dispositif capteur optique (2) selon la revendication 1, dans lequel
l'élément de support de puces (23) est un élément en feuille dont la forme est déformable.

3. Dispositif capteur optique (2) selon la revendication 1, dans lequel
la pluralité de puces de capteur optique (21₁, 21₂, 21ₖ₋₁, 21ₖ, 21_{Z-1}, 21_{Z}) sont continues via les fibres optiques (22, 22A, 22B).

4. Dispositif capteur optique (2) selon la revendication 1, dans lequel
la pluralité de puces de capteur optique (21₁, 21₂, 21ₖ₋₁, 21ₖ, 21_{Z-1}, 21_{Z}) inclut : une première puce de capteur optique (21₁) pour entrer et délivrer en sortie un signal optique vers et depuis une unité d'émetteur-récepteur optique (3, 3A, 3B) ; et une seconde puce de capteur optique (21₂) pour changer une caractéristique d'un signal optique en sortie de la puce de capteur optique (21₂) à un stade précédent et pour délivrer en sortie un signal optique avec une caractéristique changée vers la puce de capteur optique (21₂) à un stade précédent par retour, et
la première puce de capteur optique (21₁) modifie une caractéristique d'un signal optique provenant de l'unité d'émetteur-récepteur optique (3, 3A, 3B), délivre en sortie un signal optique avec une caractéristique changée vers la puce de capteur optique à un stade ultérieur, et délivre en sortie un signal optique s'étant propagé à travers la pluralité de puces de capteur optique (21₁, 21₂, 21 ₖ₋₁, 21ₖ, 21_{Z-1}, 21_{Z}) en continu entre la première puce de capteur optique (21₁) et la seconde puce de capteur optique (212) vers l'unité d'émetteur-récepteur optique (3, 3A, 3B).

5. Dispositif capteur optique (2) selon la revendication 1, dans lequel
la puce de capteur optique (21ₖ) inclut :
un guide d'ondes (2104A, 2104B) à travers lequel un signal optique se propage ;
une unité de conversion de taille de point (2101, 2103) pour convertir une taille de point d'un signal optique ; et
une ou plusieurs unités de changement (2102, 2102A à 2102C) pour changer au moins une parmi une caractéristique d'intensité, une caractéristique de phase ou une caractéristique de fréquence d'un signal optique en fonction d'un état de l'échantillon (100, 100A à 100C).

6. Dispositif capteur optique (2) selon la revendication 1, dans lequel
la pluralité de puces de capteur optique (21₁, 21₂, 21ₖ₋₁, 21ₖ, 21_{Z-1}, 21_{Z}) changent une caractéristique d'un signal optique d'entrée en fonction d'une pluralité de types d'états de l'échantillon (100, 100A à 100C).

7. Dispositif capteur optique (2) selon la revendication 4, dans lequel
la seconde puce de capteur optique (21_{Z}) inclut une unité de réflecteur (2105) pour réfléchir un signal optique avec une caractéristique changée vers la puce de capteur optique à un stade précédent.

8. Dispositif capteur optique (2) selon la revendication 5, dans lequel
la pluralité d'unités de changement (2102, 2102A à 2102B) changent une caractéristique d'un signal optique d'entrée en fonction de différents états de l'échantillon (100, 100A à 100C).

9. Dispositif capteur optique (2) selon la revendication 5, dans lequel
l'unité de conversion de taille de point (2101, 2103) est un convertisseur de taille de point ou un coupleur de réseau incluant un guide d'ondes.

10. Dispositif capteur optique (2) selon la revendication 5, dans lequel
chacune des unités de changement (2102A) est un résonateur en anneau (2106), un interféromètre de Mach-Zehnder, une combinaison du résonateur en anneau et de l'interféromètre de Mach-Zehnder, ou un élément optique pour délivrer en sortie une partie d'un signal optique se propageant à travers le guide d'ondes vers l'extérieur et pour entrer de la lumière réfléchie obtenue en réfléchissant la sortie de lumière vers l'extérieur.

11. Dispositif capteur optique (2) selon la revendication 5, dans lequel
un élément formé d'un matériau différent d'un matériau du guide d'ondes (2106) est empilé sur le guide d'ondes (2106), et
chacune des unités de changement (2102A) change une caractéristique d'un signal optique d'entrée en fonction d'un matériau de l'élément empilé sur le guide d'ondes (2106).

12. Système de mesure (1, 1A à 1C), comprenant :
le dispositif capteur optique (2) selon l'une quelconque des revendications 1 à 11,
une unité d'émetteur-récepteur optique (3, 3A, 3B) pour transmettre un signal optique à la puce de capteur optique (21₁) dans le dispositif capteur optique (2) et pour recevoir un signal optique propagé à travers la pluralité de puces de capteur optique (21₁, 21₂, 21ₖ₋₁, 21ₖ, 21_{Z-1}, 21_{Z}) prévues dans le dispositif capteur optique (2) ; et
une unité d'analyse de signal reçu (4, 75) pour mesurer un état de l'échantillon (100, 100A à 100C) en analysant un signal reçu par l'unité d'émetteur-récepteur optique (3, 3A, 3B).

13. Système de mesure (1, 1A à 1C) selon la revendication 12, comprenant en outre une unité de générateur de signal modulé (6) pour générer un signal modulé utilisé pour mesurer un état de l'échantillon (100, 100A à 100C).

14. Système de mesure (1, 1A à 1C) selon la revendication 12, dans lequel
l'unité d'analyse de signal reçu (4) spécifie chacune de positions de la pluralité de puces de capteur optique (21₁, 21₂, 21ₖ₋₁, 21ₖ, 21_{Z-1}, 21z) en utilisant un signal reçu par l'unité d'émetteur-récepteur optique (3, 3A, 3B), et mesure un état de l'échantillon (100, 100A à 100C) à chacune des positions des puces de capteur optique (21₁, 21₂, 21ₖ₋₁, 21ₖ, 21_{Z-1}, 21z).

15. Système de mesure (1, 1A à 1C) selon la revendication 13, comprenant en outre une unité de communication sans fil (76) pour effectuer une communication sans fil avec un dispositif externe (8) comprenant une unité d'affichage (83) et une unité de commande (82), dans lequel
l'unité de communication sans fil (76) amène l'unité d'affichage (83) à afficher des informations de résultat de mesure mesurées par l'unité d'analyse de signal reçu (75) en transmettant les informations de résultat de mesure au dispositif externe (8),
l'unité de commande (82) génère un signal de commande pour spécifier un état de l'échantillon (100, 100A à 100C), et
l'unité de générateur de signal modulé (73) génère un signal modulé à utiliser pour mesurer un état de l'échantillon (100, 100A à 100C) désigné par le signal de commande, et délivre en sortie le signal modulé généré vers l'unité d'émetteur-récepteur optique (71).

16. Procédé de mesure pour mesurer un état de l'échantillon (100, 100A à 100C) en utilisant le dispositif capteur optique (2) selon l'une quelconque des revendications 1 à 11, le procédé de mesure comprenant les étapes consistant à :
transmettre un signal optique à la puce de capteur optique dans le dispositif de capteur optique (2) par l'intermédiaire d'une unité d'émetteur-récepteur optique (3, 3A, 3B) ;
recevoir un signal optique propagé à travers la pluralité de puces de capteur optique (21₁, 21₂, 21ₖ₋₁, 21ₖ, 21_{Z-1}, 21_{Z}) fournies dans le dispositif capteur optique (2) par l'unité d'émetteur-récepteur optique (3, 3A, 3B) ; et
mesurer un état de l'échantillon (100, 100A à 100C) en analysant un signal reçu par l'unité d'émetteur-récepteur optique (3, 3A, 3B), par l'intermédiaire d'une unité d'analyse de signal reçu (4).
